# EUROPEAN PATENT APPLICATION

(11) **EP 4 324 450 A1**
(43) Date of publication of application: **21.02.2024**
(21) Application number: 22788173.7
(22) Date of filing: 12.04.2022
(51) Int. Cl.: A61K 6/00, A61K 6/60, A61K 6/831, A61K 6/838

(54) **DENTAL FILLING KIT**

(30) Priority: 13.04.2021 JP 2021067662
(71) Applicant: Kuraray Noritake Dental Inc., Kurashiki-shi, Okayama 710-0801 (JP)
(72) Inventor: MATSUURA, Ryo, Tainai-shi, Niigata 959-2653 (JP); NOJIRI, Yamato, Tainai-shi, Niigata 959-2653 (JP)
(74) Representative: D Young & Co LLP
(86) International application number: PCT/JP2022/017645
(87) International publication number: WO 2022/220250

(57) **Abstract**

The present invention provides a dental filling kit that comprises a dental cleaning material and a self-adhesive dental composite resin, and that exhibits good adhesive properties to tooth structure, and good sealing properties for cavities. The present invention relates to a dental filling kit comprising: a self-adhesive dental composite resin (X) comprising a monomer (a) having an acidic group, a monomer (b) having no acidic group, a polymerization initiator (c), and a filler (d); and a dental cleaning material (Y) having a pH of 2.0 or more and less than 9.0.

## Description

### TECHNICAL FIELD

The present invention relates to dental filling kits. More specifically, the present invention relates to a dental filling kit that comprises a dental cleaning material and a self-adhesive dental composite resin, and that exhibits good adhesive properties to tooth structure, and good sealing properties for cavities after cleaning tooth surfaces with the dental cleaning material, and filling the cavities with the self-adhesive dental composite resin.

### BACKGROUND ART

Restorative filling materials such as filling composite resins and filling compomers, and crown restoration materials such as metal alloys, porcelain, and resin materials are typically used for the restoration of damaged tooth structures (enamel, dentin, and cementum) caused by caries and other dental defects. However, restorative filling materials and crown restoration materials (in this specification, these are also referred to collectively as "dental restoration materials") themselves do not generally possess adhesive properties to the tooth structure. Consequently, various adhesive systems with bonding agents have been used for the bonding of the tooth structure and dental restoration materials. An example of adhesive systems that are in common use is the acid etching (total etching) adhesive system, whereby the surfaces of tooth structure is etched with an acid etchant such as a phosphoric acid aqueous solution, and a bonding material, or an adhesive, is applied to bond a dental restoration material to the tooth structure.

The self-etching adhesive system, on the other hand, is an example of adhesive systems that do not use acid etchants. In the past, the mainstream in such adhesive systems has been a two-step process in which a self-etching primer containing an acidic monomer, a hydrophilic monomer, and water is first applied to surfaces of tooth structure, and a bonding material containing a crosslinkable monomer and a polymerization initiator is applied without rinsing the surfaces with water. Another type of self-etching adhesive system that has come to be commonly used in recent years is the single-step adhesive system, which uses a one-part dental adhesive (one-part bonding material) that serves as both a self-etching primer and a bonding material. The typical monomer components of the one-part bonding material are acidic monomers, hydrophilic monomers, and crosslinkable monomers, and the one-part bonding material typically uses water or hydrophilic volatile organic solvents.

Recent years have seen the development of self-adhesive dental composite resins possessing adhesive properties in the dental composite resins themselves, and some of the compositions that have already been put into practical use eliminate the use of bonding materials to reduce the number of steps in restorative treatment procedures (Patent Literatures 1 to 4).

There has also been development of dental cleaning materials for washing purposes against contamination caused by protein-containing bodily fluids on dental restorations and abutment teeth such as tooth surfaces (Patent Literatures 5 to 7).

### CITATION LIST

### Patent Literature

Patent Literature 1: JP 2004-529946 T
Patent Literature 2: JP 2017-105716 A
Patent Literature 3: JP 2018-065831 A
Patent Literature 4: WO2015/190101
Patent Literature 5: JP H04-1114 A
Patent Literature 6: WO2020/105642
Patent Literature 7: JP 2020-083793 A

### SUMMARY OF INVENTION

### Technical Problem

However, the self-adhesive dental materials and self-adhesive dental composite resins described in Patent Literatures 1 to 4 involve the use of fine silicon carbide sand paper, #1000 silicon carbide paper, and #600 emery paper for the evaluation of adhesive properties to tooth structure, leaving a bonding surface with trace amounts of debris, or a smear layer as it is also called. The smear layer is an unstructured layer with debris of mineralized tissue, and removal of the smear layer is thought to be crucial for enabling bonding with dentin.

On the other hand, a thick smear layer results when deep cavities are formed with a diamond bur, an instrument actually used to form cavities as clinically practiced. Studies conducted by the present inventors revealed that, contrary to the total-etching and self-etching bonding materials that allow for formation of a bonding surface with sparse smear layer presence by the effect of acid and water dissolving the smear layer, the self-adhesive dental composite resins, as a water-free composition, do not possess the smear-layer dissolving effect by acid and water, failing to facilitate demineralization of the smear layer, and to provide a sufficient bonding area for the tooth structure, particularly the dentin. It was found that this may lead to a failure to provide sufficient bond strength, and can result in deterioration of marginal sealing properties around the cavities (hereinafter, also referred to as "cavity sealing properties").

Another finding concerning cavity sealing properties is that, because of the polymerization shrinkage stress of the self-adhesive dental composite resin, the composite resin after the polymerization and curing of the self-adhesive dental composite resin filled into the cavity may detach itself from the cavity floor forming the cavity. Bonding is particularly difficult when the cavity floor lies within the dentin because the dentin is directly affected by the moisture supplied through the tooth pulp.

In photopolymerizable self-adhesive dental composite resins, the resin filling the cavities starts polymerizing in portions closer to the light source above the cavities, and polymerization shrinkage acts to pull the self-adhesive dental composite resin filling away from the cavity floor. That is, bonding to the dentin at the cavity floor is most demanding and particularly challenging in the restoration of cavities using photopolymerizable self-adhesive dental composite resins.

Patent Literature 5 discloses applying a dental filler kit that comprises a dental adhesive composite resin, after washing the tooth structure with a dental cleaning material. However, the dental filler kit of Patent Literature 5 is a product composed of an etchant, a bonding material, and a chemically polymerizable dental adhesive composite resin, with the etchant containing phosphoric acid and water, and producing the acid-water effect. Patent Literature 5 indicates that there is no significant difference in adhesive properties to dentin compared to enamel, for which improved adhesive properties are said to be obtained when the tooth structure is rinsed with a dental cleaning material.

As a measure of adhesive properties, Patent Literatures 6 to 7 evaluate the bond durability of dental resin cements to dental restoration zirconia after washing with a dental cleaning material. However, the evaluation does not relate to self-adhesive dental composite resins, and no evaluation is made with respect to the adhesive properties of self-adhesive dental composite resins to tooth structure.

It was also found through investigations by the present inventors that, because of poor demineralization capability and lower penetrability compared to bonding materials that exhibit excellent penetrability by containing water, self-adhesive dental composite resins are susceptible to contamination from saliva, blood, and other such substances during treatment, leading to a significant decrease in bond strength to tooth structure.

As discussed above, self-adhesive dental composite resins involve specific challenges due to their unique compositions and clinical practice.

It is an object of the present invention to provide a dental filling kit that comprises a dental cleaning material and a self-adhesive dental composite resin, and that exhibits good adhesive properties to tooth structure, and good sealing properties for cavities.

### Solution to Problem

The present inventors conducted intensive studies, and found that the foregoing issues can be solved with a dental filling kit that comprises a dental cleaning material of a specific composition, and a self-adhesive dental composite resin of a specific composition. The present invention was completed after further studies.

Specifically, the present invention includes the following.
[1] A dental filling kit comprising:
   a self-adhesive dental composite resin (X) comprising a monomer (a) having an acidic group, a monomer (b) having no acidic group, a polymerization initiator (c), and a filler (d); and
   a dental cleaning material (Y) having a pH of 2.0 or more and less than 9.0.
[2] The dental filling kit according to [1], wherein the dental cleaning material (Y) comprises a cationic component (f), an anionic component (g), and water (h).
[3] The dental filling kit according to [2], wherein the anionic component (g) comprises a monomer (g-1) having an acidic group.
[4] The dental filling kit according to [3], wherein the monomer (g-1) having an acidic group comprises a monomer having a phosphoric acid group.
[5] The dental filling kit according to [3] or [4], wherein the monomer (g-1) having an acidic group comprises a monomer having a divalent phosphoric acid group with a C6 to C12 alkylene group.
[6] The dental filling kit according to any one of [3] to [5], wherein the monomer (g-1) having an acidic group comprises a compound identical to the monomer (a) having an acidic group.
[7] The dental filling kit according to any one of [2] to [6], wherein the cationic component (f) comprises a basic compound.
[8] The dental filling kit according to [7], wherein the basic compound comprises an organic amine compound (f-1).
[9] The dental filling kit according to [8], wherein the organic amine compound (f-1) comprises a tertiary organic amine compound.
[10] The dental filling kit according to [9], wherein the tertiary organic amine compound comprises triethanolamine.
[11] The dental filling kit according to any one of [2] to [10], wherein the content of the water (h) is 40 to 99.8 parts by mass relative to 100 parts by mass of the dental cleaning material (Y).
[12] The dental filling kit according to any one of [1] to [11], wherein the dental cleaning material (Y) has a pH of 2.0 or more and less than 8.0.
[13] The dental filling kit according to any one of [1] to [12], wherein the content of the filler (d) is 50 to 90 parts by mass in total 100 parts by mass of the self-adhesive dental composite resin (X).
[14] The dental filling kit according to any one of [1] to [13], wherein the dental cleaning material (Y) is essentially free of the monomer (b) having no acidic group.
[15] The dental filling kit according to any one of [1] to [14], wherein the dental cleaning material (Y) is essentially free of a polymerization initiator.
[16] The dental filling kit according to any one of [1] to [15], wherein the polymerization initiator (c) comprises a photopolymerization initiator (c-1).

### Advantageous Effects of Invention

According to the present invention, a dental filling kit can be provided that comprises a dental cleaning material and a self-adhesive dental composite resin, and that exhibits good adhesive properties to tooth structure, and good sealing properties for cavities.

According to the present invention, a dental filling kit can be provided that comprises a dental cleaning material and a self-adhesive dental composite resin, and that does not limit the composition of the self-adhesive dental composite resin so that good cavity sealing properties can be achieved even when a photopolymerizable self-adhesive dental composite resin is used.

### DESCRIPTION OF EMBODIMENTS

A dental filling kit of the present invention comprises a self-adhesive dental composite resin (X) and a dental cleaning material (Y). The dental cleaning material (Y) has a pH of 2.0 or more and less than 9.0. The self-adhesive dental composite resin (X) comprises a monomer (a) having an acidic group, a monomer (b) having no acidic group, a polymerization initiator (c), and a filler (d). A dental filling kit of the present invention is used by filling the self-adhesive dental composite resin (X) after applying the dental cleaning material (Y) within the cavities.

In this specification, "(meth)acryl" is a collective term for methacryl and acryl. The same applies to similar expressions, such as "(meth)acrylic acid" and "(meth)acrylonitrile". In the present specification, the upper limits and lower limits of numeric ranges (for example, ranges of contents of components, ranges of values calculated from components, and ranges of physical properties) can be appropriately combined.

It remains unclear why a dental filling kit of the present invention exhibits good adhesive properties to tooth structure and good sealing properties for cavities. However, the following speculation has been made.

Bonding is possible without impairing the adhesive properties of the self-adhesive dental composite resin (X), possibly as a result of the dental cleaning material (Y) removing the debris-containing smear layer created when forming cavities (hereinafter, also referred to as "smear layer "), and eliminating contamination caused by protein-containing bodily fluids, without altering the state of the tooth structure at the bonding surface. With the present invention, a decrease in adhesive properties can be reduced because the dental cleaning material (Y) hardly demineralizes the tooth structure itself while enabling removal of the smear layer.

The following describes the components used in a self-adhesive dental composite resin (X) of the present invention.

### <Monomer (a) Having Acidic Group>

In view of adhesive properties to tooth structure, a monomer (a) having an acidic group is essential in a self-adhesive dental composite resin (X) of the present invention. Preferred for use in the self-adhesive dental composite resin (X) are radical monomers. Specific examples of radical monomers in monomer (a) having an acidic group include (meth)acrylate monomers, (meth)acrylamide monomers, esters, vinyl esters, and vinyl ethers of acids such as α-cyanoacrylic acid, (meth)acrylic acid, α-halogenated acrylic acid, crotonic acid, cinnamic acid, sorbic acid, maleic acid, and itaconic acid, and mono-N-vinyl derivatives and styrene derivatives. In view of curability, preferred are (meth)acrylate monomers and (meth)acrylamide monomers.

Examples of the monomer (a) having an acidic group used in the present invention include monomers having at least one acidic group such as a phosphoric acid group, a pyrophosphoric acid group, a thiophosphoric acid group, a phosphonic acid group, a carboxylic acid group, and a sulfonic acid group. The monomer (a) having an acidic group may be used alone, or two or more thereof may be used in appropriate combinations. Specific examples of the monomer (a) having an acidic group are as follows.

Examples of monomers having a phosphoric acid group include 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethylphenyl hydrogen phosphate, 2-(meth)acryloyloxyethyl-(2-bromoethyl)hydrogen phosphate, 2-methacryloyloxyethyl-(4-methoxyphenyl)hydrogen phosphate, 2-methacryloyloxypropyl-(4-methoxyphenyl)hydrogen phosphate, and acid chlorides, alkali metal salts, and amine salts of these.

Examples of monomers having a pyrophosphoric acid group include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and acid chlorides, alkali metal salts, and amine salts of these.

Examples of monomers having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyicosyl dihydrogen thiophosphate, and acid chlorides, alkali metal salts, and ammonium salts of these.

Examples of monomers having a phosphonic acid group include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexylphosphonoacetate, 10-(meth)acryloyloxydecylphosphonoacetate, and acid chlorides, alkali metal salts, and ammonium salts of these.

Examples of monomers having a carboxylic acid group include monofunctional (meth)acrylic acid esters having one carboxyl group or acid anhydride group thereof within the molecule, and monofunctional (meth)acrylic acid esters having a plurality of carboxyl groups or acid anhydride groups thereof within the molecule.

Examples of monofunctional monomers having one carboxyl group or acid anhydride group thereof within the molecule include (meth)acrylic acid, N-(meth)acryloylglycine, N-(meth)acryloylaspartic acid, 2-(meth)acryloyloxyethyl hydrogen succinate, 2-(meth)acryloyloxyethyl hydrogen phthalate, 2-(meth)acryloyloxyethyl hydrogen malate, O-(meth)acryloyltyrosine, N-(meth)acryloyltyrosine, N-(meth)acryloylphenylalanine, N-(meth)acryloyl-p-aminobenzoic acid, N-(meth)acryloyl-o-aminobenzoic acid, 2-(meth)acryloyloxybenzoic acid, 3-(meth)acryloyloxybenzoic acid, 4-(meth)acryloyloxybenzoic acid, N-(meth)acryloyl-5-aminosalicylic acid, and N-(meth)acryloyl-4-aminosalicylic acid, and compounds derived by converting the carboxyl group of the aforementioned compounds into an acid anhydride group.

Examples of monofunctional monomers having a plurality of carboxyl groups or acid anhydride groups thereof within the molecule include 6-(meth)acryloyloxyhexane-1,1 -dicarboxylic acid, 9-(meth)acryloyloxynonane-1,1 -dicarboxylic acid, 10-(meth)acryloyloxydecane-1, 1 -dicarboxylic acid, 11-(meth)acryloyloxyundecane-1,1 - dicarboxylic acid, 12-(meth)acryloyloxydodecane-1, 1 -dicarboxylic acid, 13-(meth)acryloyloxytridecane-1,1-dicarboxylic acid, 4-(meth)acryloyloxyethyltrimellitate, 4-(meth)acryloyloxyethyltrimellitate anhydride, 4-(meth)acryloyloxybutyltrimellitate, 4-(meth)acryloyloxyhexyltrimellitate, 4-(meth)acryloyloxydecyltrimellitate, 2-(meth)acryloyloxyethyl-3'-(meth)acryloyloxy-2'-(3,4-dicarboxybenzoyloxy)propylsuccinate, 6-(meth)acryloyloxyethylnaphthalene-1,2,6-tricarboxylic acid anhydride, 6-(meth)acryloyloxyethylnaphthalene-2,3,6-tricarboxylic acid anhydride, 4-(meth)acryloyloxyethylcarbonylpropionoyl-1,8-naphthalic acid anhydride, and 4-(meth)acryloyloxyethylnaphthalene-1 ,8-tricarboxylic acid anhydride.

Examples of monomers having a sulfonic acid group include 2-sulfoethyl (meth)acrylate.

In view of providing good bond strength when the monomer (a) having an acidic group is used as a component of the self-adhesive dental composite resin (X), preferred among the foregoing monomers (a) having an acidic group are monomers having a phosphoric acid group, or monomers having a carboxylic acid group, more preferably 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, 4-(meth)acryloyloxyethyltrimellitate anhydride, 4-(meth)acryloyloxyethyltrimellitate, 11-(meth)acryloyloxyundecane-1,1-dicarboxylic acid, or a mixture of 2-methacryloyloxyethyl dihydrogen phosphate and bis(2-methacryloyloxyethyl)hydrogen phosphate, even more preferably 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, or 20-(meth)acryloyloxyicosyl dihydrogen phosphate. In view of a balance with curability, 10-(meth)acryloyloxydecyl dihydrogen phosphate is particularly preferred.

In view of adhesive properties to tooth structure, the content of the monomer (a) having an acidic group in a self-adhesive dental composite resin (X) of the present invention is preferably 1 to 40 parts by mass, more preferably 2.5 to 35 parts by mass, even more preferably 5 to 30 parts by mass in total 100 parts by mass of monomers in a self-adhesive dental composite resin (X) of the present invention.

### <Monomer (b) Having no Acidic Group>

Examples of the monomer (b) having no acidic group in the present invention include asymmetrical acrylamide methacrylic acid ester compounds (b-1); hydrophobic monomers (b-2) having no acidic group and having a solubility of less than 10 mass% in 25°C water (hereinafter, also referred to simply as "hydrophobic monomers (b-2)"); and hydrophilic monomers (b-3) having no acidic group and having a solubility of 10 mass% or more in 25°C water (hereinafter, also referred to simply as "hydrophilic monomers (b-3)"). The monomer (b) having no acidic group may be used alone, or two or more thereof may be used in combination. In the present invention, compounds having no acidic group and containing an acrylamide group and a methacryloyloxy group are classified as asymmetrical acrylamide-methacrylic acid ester compounds (b-1), whereas compounds having no acidic group and excluded from asymmetrical acrylamide methacrylic acid ester compounds (b-1) are classified as hydrophobic monomers (b-2) or hydrophilic monomers (b-3), depending on the degree of hydrophilicity.

### · Asymmetrical Acrylamide·Methacrylic Acid Ester Compound (b-1)

A certain preferred embodiment is, for example, a self-adhesive dental composite resin (X) comprising an asymmetrical acrylamide methacrylic acid ester compound (b-1). In view of improvement of properties such as adhesive properties to tooth structure, the asymmetrical acrylamide-methacrylic acid ester compound (b-1) is preferably a compound represented by the following general formula (1). 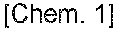 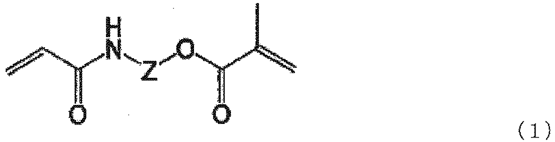 In the formula, Z is an optionally substituted C₁ to C₈ linear or branched aliphatic group or an optionally substituted aromatic group, and the aliphatic group may be interrupted with at least one binding group selected from the group consisting of -O-, -S-, -CO-, -CO-O-, - O-CO-, -NR¹-, -CO-NR'-, -NR'-CO-, -CO-O-NR'-, -O-CO-NR¹-, and -NR'-CO-NR'-. R¹ represents a hydrogen atom, or an optionally substituted C₁ to C₈ linear or branched aliphatic group.

Z is a moiety that adjusts the hydrophilicity of the asymmetrical acrylamide methacrylic acid ester compound (b-1). The optionally substituted C₁ to C₈ aliphatic group represented by Z may be either a saturated aliphatic group (an alkylene group, a cycloalkylene group (for example, such as a 1 ,4-cyclohexylene group)), or an unsaturated aliphatic group (an alkenylene group, an alkynylene group). In view of availability or ease of production, and chemical stability, preferred are saturated aliphatic groups (alkylene groups). In view of adhesive properties to tooth structure and polymerization curability, Z is preferably an optionally substituted linear or branched C₁ to C₄ aliphatic group, more preferably an optionally substituted linear or branched C₂ to C₄ aliphatic group. The aliphatic group is preferably an alkylene group. Examples of the C₁ to C₈ alkylene group include a methylene group, an ethylene group, an n-propylene group, an isopropylene group, and an n-butylene group.

Examples of the optionally substituted aromatic group represented by Z include an arylene group, and an aromatic heterocyclic group. The aromatic group is preferably an arylene group rather than an aromatic heterocyclic group. The hetero ring in the aromatic heterocyclic group is normally unsaturated. The aromatic hetero ring is preferably a five-membered ring or a six-membered ring. Preferably, the arylene group is, for example, a phenylene group. Examples of the hetero ring in the aromatic heterocyclic group include a furan ring, a thiophene ring, a pyrrole ring, an oxazole ring, an isoxazole ring, a thiazole ring, an isothiazole ring, an imidazole ring, a pyrazole ring, a furazan ring, a triazole ring, a pyran ring, a pyridine ring, a pyridazine ring, a pyrimidine ring, a pyrazine ring, and a 1,3,5-triazine ring. The aromatic group is particularly preferably a phenylene group.

The aliphatic group represented by R¹ may be either a saturated aliphatic group (an alkyl group) or an unsaturated aliphatic group (an alkenyl group, an alkynyl group). In view of availability or ease of production, and chemical stability, preferred are saturated aliphatic groups (alkyl groups). Examples of the C₁ to C₈ linear or branched alkyl group represented by R¹ include methyl, ethyl, n-propyl, isopropyl, n-butyl, isobutyl, sec-butyl, tert-butyl, n-pentyl, isopentyl, sec-pentyl, neopentyl, tert-pentyl, 1-ethylpropyl, hexyl, isohexyl, 1,1-dimethylbutyl, 2,2-dimethylbutyl, 3,3-dimethylbutyl, and 2-ethylbutyl. Preferred examples include methyl, ethyl, n-propyl, isopropyl, n-butyl, isobutyl, sec-butyl, and tert-butyl.

R¹ is more preferably a hydrogen atom, or an optionally substituted linear or branched C₁ to C₄ alkyl group, even more preferably a hydrogen atom, or an optionally substituted linear or branched C₁ to C₃ alkyl group.

When the aliphatic group represented by Z is interrupted by the above binding group, the number of binding groups is not particularly limited, and may be about 1 to 10, preferably 1, 2, or 3, more preferably 1 or 2. In the formula (1), the aliphatic group represented by Z is preferably one that is not contiguously interrupted by the binding group. That is, the aliphatic group represented by Z is preferably one in which the binding groups are not adjacent to each other. The binding group is more preferably at least one selected from the group consisting of -O-, -S-, -CO-, -CO-O-, -O-CO-, -NH-, - CO-NH-, -NH-CO-, -CO-O-NH-, -O-CO-NH-, and -NH-CO-NH-, particularly preferably at least one selected from the group consisting of -O-, -S-, -CO-, -NH-, -CO-NH-, and -NH-CO-.

Examples of the substituents in Z include halogen atoms (a fluorine atom, a chlorine atom, a bromine atom, an iodine atom), a carboxyl group, a C₂ to C₆ linear or branched acyl group, a C₁ to C₆ linear or branched alkyl group, and a C₁ to C₆ linear or branched alkoxy group.

Specific examples of the asymmetric acrylamide methacrylic acid ester compound (b-1) include, but are not particularly limited to, the following.

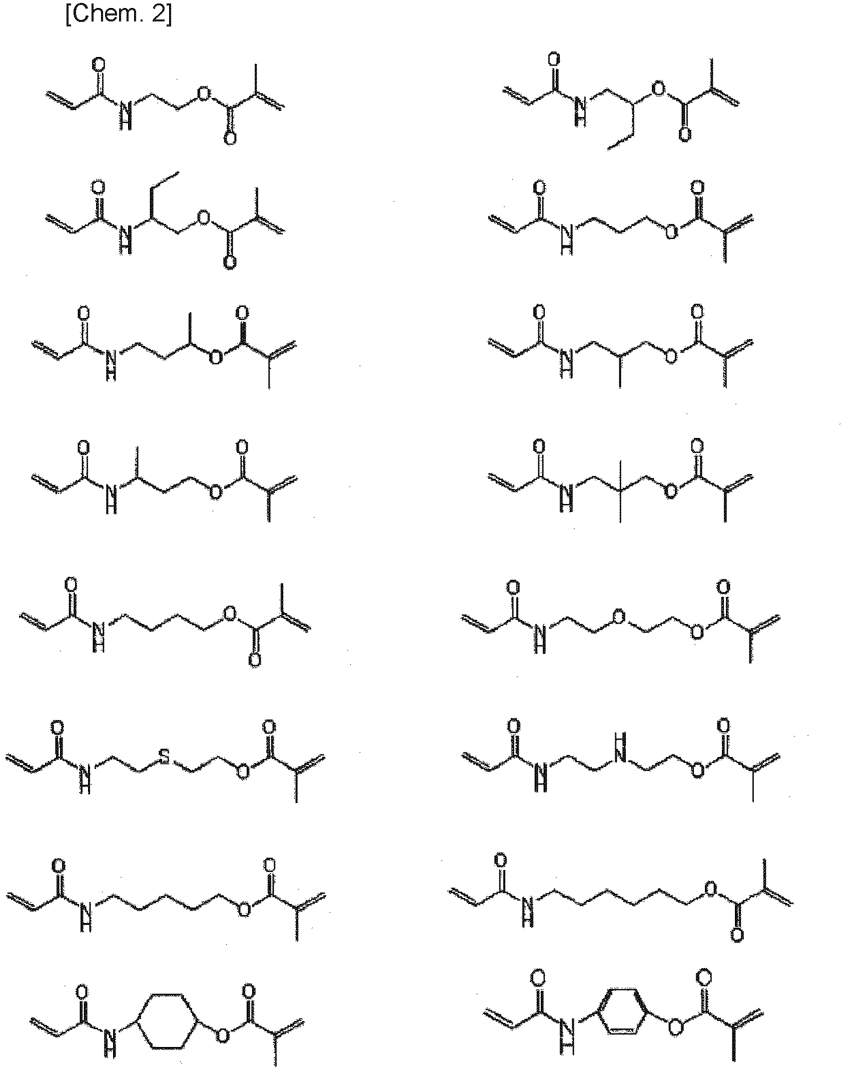

In view of adhesive properties to tooth structure and polymerization curability, preferred are asymmetric acrylamide-methacrylic acid ester compounds in which Z is an optionally substituted C₂ to C₄ linear or branched aliphatic group, more preferably N-methacryloyloxyethylacrylamide (commonly known as MAEA), N-methacryloyloxypropylacrylamide, N-methacryloyloxybutylacrylamide, N-(1-ethyl-(2-methacryloyloxy)ethyl)acrylamide, and N-(2-(2-methacryloyloxyethoxy)ethyl)acrylamide. In view of the high hydrophilicity concerning penetration into the collagen layer of dentin, even more preferred are MAEA, and N-methacryloyloxypropylacrylamide.

The asymmetric acrylamide-methacrylic acid ester compound (b-1) may be contained alone, or two or more thereof may be contained in combination. The content of asymmetric acrylamide-methacrylic acid ester compound (b-1) is not particularly limited, as long as the present invention can exhibit its effects. However, the content of asymmetric acrylamide-methacrylic acid ester compound (b-1) is preferably 1 to 60 parts by mass, more preferably 2 to 45 parts by mass, even more preferably 3 to 30 parts by mass, particularly preferably 5 to 25 parts by mass in total 100 parts by mass of monomers in a self-adhesive dental composite resin (X) of the present invention.

### · Hydrophobic Monomer (b-2) Having no Acidic Group

The hydrophobic monomer (b-2) having no acidic group improves properties such as ease of handling of the self-adhesive dental composite resin (X), and the mechanical strength of the cured product. The hydrophobic monomer (b-2) is preferably a radical monomer having no acidic group but having a polymerizable group. In view of ease of radical polymerization, preferred as the polymerizable group are (meth)acryl groups and/or (meth)acrylamide groups. The hydrophobic monomer (b-2) means a monomer that has no acidic group, and does not correspond to the asymmetric acrylamide-methacrylic acid ester compound (b-1), and that has a solubility of less than 10 mass% in 25°C water. Examples of the hydrophobic monomer (b-2) include crosslinkable monomers such as aromatic bifunctional monomers, aliphatic bifunctional monomers, and tri- and higher-functional monomers.

Examples of the aromatic bifunctional monomers include 2,2-bis((meth)acryloyloxyphenyl)propane, 2,2-bis[4-(3-(meth)acryloyloxy-2-hydroxypropoxy)phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, and 2,2-bis(4-(meth)acryloyloxyisopropoxyphenyl)propane. Preferred are 2,2-bis[4-(3-methacryloyloxy-2-hydroxypropoxy)phenyl]propane (commonly known as Bis-GMA), 2,2-bis(4-(meth)acryloyloxyethoxyphenyl)propane, 2,2-bis(4-methacryloyloxypolyethoxyphenyl)propane (with an average of 2.6 moles of ethoxy group added, commonly known as D-2.6E), 2,2-bis(4-(meth)acryloyloxydiethoxyphenyl)propane, 2,2-bis(4-(meth)acryloyloxytriethoxyphenyl)propane, 2,2-bis(4-(meth)acryloyloxytetraethoxyphenyl)propane, and 2,2-bis(4-(meth)acryloyloxypentaethoxyphenyl)propane.

Examples of the aliphatic bifunctional monomers include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,2-bis(3-methacryloyloxy-2-hydroxypropoxy)ethane, and 2,2,4-trimethylhexamethylenebis(2-carbamoyloxyethyl)di(meth)acrylate. Preferred are triethylene glycol diacrylate, triethylene glycol dimethacrylate (commonly known as 3G), neopentyl glycol di(meth)acrylate, 1,2-bis(3-methacryloyloxy-2-hydroxypropoxy)ethane, 2,2,4-trimethylhexamethylenebis(2-carbamoyloxyethyl)dimethacrylate (commonly known as UDMA), and 1,10-decanediol dimethacrylate (commonly known as DD).

Examples of the tri- and higher-functional monomers include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, N,N-(2,2,4-trimethylhexamethylene)bis[2-(aminocarboxy)propane-1,3-diol]tetra(meth)acrylate, and 1,7-diacryloyloxy-2,2,6,6-tetra(meth)acryloyloxymethyl-4-oxaheptane. Preferred is N,N-(2,2,4-trimethylhexamethylene)bis[2-(aminocarboxy)propane-1,3-diol]tetramethacrylate.

In view of mechanical strength and ease of handling, the preferred hydrophobic monomers (b-2) are aromatic bifunctional monomers, and aliphatic bifunctional monomers. Preferred as the aromatic bifunctional monomers are Bis-GMA and D-2.6E. Preferred as the aliphatic bifunctional monomers are glycerol di(meth)acrylate, 3G, neopentyl glycol di(meth)acrylate, 1,6-hexanediol di(meth)acrylate, DD, 1,2-bis(3-methacryloyloxy-2-hydroxypropoxy)ethane, and UDMA.

In view of providing good adhesive properties to tooth structure when the hydrophobic monomer (b-2) is used as a component of the self-adhesive dental composite resin (X) (composition), the hydrophobic monomer (b-2) is more preferably Bis-GMA, D-2.6E, 3G, UDMA, or DD, even more preferably D-2.6E, 3G, or Bis-GMA.

The hydrophobic monomer (b-2) may be contained alone, or two or more thereof may be used in combination. When the content of hydrophobic monomer (b-2) is at or below the upper limits specified below, a decrease of adhesion due to reduced wettability of the self-adhesive dental composite resin (X) to tooth structure can more easily be reduced, whereas the cured product can more easily exhibit the desired mechanical strength when the content of hydrophobic monomer (b-2) is at or above the lower limits specified below The content of the hydrophobic monomer (b-2) in a self-adhesive dental composite resin (X) of the present invention is preferably 20 to 98 parts by mass, more preferably 40 to 95 parts by mass, even more preferably 60 to 92 parts by mass in total 100 parts by mass of monomers in a self-adhesive dental composite resin (X) of the present invention.

### · Hydrophilic Monomer (b-3) Having no Acidic Group

The hydrophilic monomer (b-3) improves the wettability of self-adhesive dental composite resin (X) to tooth structure. The hydrophilic monomer (b-3) is preferably a radical monomer having no acidic group but having a polymerizable group. In view of ease of radical polymerization, preferred as the polymerizable group are (meth)acryl groups and/or (meth)acrylamide groups. The hydrophilic monomer (b-3) means a monomer that has no acidic group, and does not correspond to the asymmetric acrylamide-methacrylic acid ester compound (b-1), and that has a solubility of 10 mass% or more in 25°C water. The hydrophilic monomer (b-3) is preferably one having a solubility of 30 mass% or more in 25°C water, more preferably one that can dissolve in water in any proportions at 25°C. The hydrophilic monomer is preferably one having a hydrophilic group such as a hydroxyl group, an oxymethylene group, an oxyethylene group, an oxypropylene group, or an amide group. Examples of the hydrophilic monomer (b-3) include hydrophilic monofunctional (meth)acrylate monomers such as 2-hydroxyethyl (meth)acrylate, 3-hydroxypropyl (meth)acrylate, 2-hydroxypropyl (meth)acrylate, 1,3-dihydroxypropyl (meth)acrylate, 2,3-dihydroxypropyl (meth)acrylate, 2-((meth)acryloyloxy)ethyltrimethylammonium chloride, and polyethylene glycol di(meth)acrylate (with nine or more oxyethylene groups); and hydrophilic monofunctional (meth)acrylamide monomers such as N-methylol(meth)acrylamide, N-hydroxyethyl(meth)acrylamide, N,N-bis(2-hydroxyethyl)(meth)acrylamide, N-methoxymethyl(meth)acrylamide, N-ethoxymethyl(meth)acrylamide, diacetone(meth)acrylamide, 4-(meth)acryloylmorpholine, N-trihydroxymethyl-N-methyl(meth)acrylamide, N,N-dimethylacrylamide, and N,N-diethylacrylamide.

In view of adhesive properties to tooth structure, preferred as hydrophilic monomer (b-3) are 2-hydroxyethyl (meth)acrylate, 2,3-dihydroxypropyl (meth)acrylate, and hydrophilic monofunctional (meth)acrylamide monomers, more preferably 2-hydroxyethyl (meth)acrylate, N,N-dimethylacrylamide, and N,N-diethylacrylamide. The hydrophilic monomer (b-3) may be contained alone, or two or more thereof may be used in combination.

The content of the hydrophilic monomer (b-3) in a self-adhesive dental composite resin (X) of the present invention ranges preferably from 0 to 50 parts by mass, more preferably from 0 to 40 parts by mass, even more preferably from 0 to 30 parts by mass in total 100 parts by mass of monomers. The content of hydrophilic monomer (b-3) may be 0 part by mass in total 100 parts by mass of monomers. When the content of the hydrophilic monomer (b-3) in a self-adhesive dental composite resin (X) of the present invention is at or above these lower limits, it is easier to produce a sufficient adhesion improving effect, whereas the cured product can more easily exhibit the desired mechanical strength when the content of hydrophilic monomer (b-3) is at or below the foregoing upper limits.

The content of the monomer (b) having no acidic group in the self-adhesive dental composite resin (X) is preferably 60 to 99 parts by mass, more preferably 65 to 97.5 parts by mass, even more preferably 70 to 95 parts by mass in total 100 parts by mass of monomers. In view of adhesive properties to tooth structure, the hydrophobic monomer (b-2) and hydrophilic monomer (b-3) have a mass ratio (hydrophobic monomer (b-2):hydrophilic monomer (b-3)) of preferably 10:0 to 1:2, more preferably 10:0 to 1:1, even more preferably 10:0 to 2:1.

A certain preferred embodiment is, for example, a self-adhesive dental composite resin (X) that is essentially free of a bi- or higher functional (meth)acrylamide monomer. Another certain preferred embodiment is, for example, a self-adhesive dental composite resin (X) that is essentially free of a phosphoric acid hydrogen diester group-containing monomer. Preferably, the phosphoric acid hydrogen diester group-containing monomer has a (meth)acryloyloxy group and/or a (meth)acrylamide group. In the present invention, "being essentially free of a monomer" means that the content of the monomer is less than 0.5 parts by mass, preferably less than 0.1 parts by mass, more preferably less than 0.01 parts by mass in total 100 parts by mass of monomers contained in a composition of the self-adhesive dental composite resin (X). Here, the monomer content may be 0 part by mass. The content of the monomer that is essentially absent may be less than 0.5 mass%, or less than 0.1 mass% in the whole composition of the self-adhesive dental composite resin (X).

### <Polymerization Initiator (c)>

The polymerization initiator (c) may be a photopolymerization initiator (c-1) or a chemical polymerization initiator (c-2). These may be contained alone, or may be used in combination.

The photopolymerization initiator (c-1) can be classified into a water-soluble photopolymerization initiator (c-1a) and a water-insoluble photopolymerization initiator (c-1b). The photopolymerization initiator (c-1) may be solely a water-soluble photopolymerization initiator (c-1a) or a water-insoluble photopolymerization initiator (c-1b), or may be a combination of a water-soluble photopolymerization initiator (c-1a) and a water-insoluble photopolymerization initiator (c-1b). Preferably, a water-soluble photopolymerization initiator (c-1a) and a water-insoluble photopolymerization initiator (c-1b) are used in combination.

### · Water-Soluble Photopolymerization Initiator (c-1a)

With a water-soluble photopolymerization initiator (c-1a), a high bond strength can be achieved by improving polymerization curability at the interface with hydrophilic tooth surfaces. The water-soluble photopolymerization initiator (c-1a) has a solubility in 25°C water of 10 g/L or more, preferably 15 g/L or more, more preferably 20 g/L or more, even more preferably 25 g/L or more. By having a solubility of 10 g/L or more in 25°C water, the water-soluble photopolymerization initiator (c-1a) can sufficiently dissolve in water within the tooth structure at the bonding interface, and more easily exhibits the polymerization promoting effect.

Examples of the water-soluble photopolymerization initiator (c-1a) include water-soluble thioxanthones; water-soluble acylphosphine oxides; and α-hydroxyalkylacetophenones, for example, such as compounds having a (poly)ethylene glycol chain introduced into the hydroxyl group(s) of 1-[4-(2-hydroxyethoxy)-phenyl]-2-hydroxy-2-methyl-1-propan-1-one, compounds having a (poly)ethylene glycol chain introduced into the hydroxyl group and/or phenyl group of 1-hydroxycyclohexyl phenyl ketone, compounds having -OCH₂COO⁻Na⁺ introduced into the phenyl group of 1-hydroxycyclohexyl phenyl ketone, compounds having a (poly)ethylene glycol chain introduced into the hydroxyl group and/or phenyl group of 2-hydroxy-2-methyl-1-phenylpropan-1-one, and compounds having -OCH₂COO⁻Na⁺ introduced into the phenyl group of 2-hydroxy-2-methyl-1-phenylpropan-1-one. Other examples of the water-soluble photopolymerization initiator (c-1a) include quaternary ammonium compounds prepared by quaternization of the amino group of α-aminoalkylphenones, such as 2-methyl-1-[4-(methylthio)phenyl]-2-morpholinopropan-1-one, and 2-benzyl-2-(dimethylamino)-1--[(4-morpholino)phenyl]-1-butanon.

The water-soluble thioxanthones may be any of, for example, 2-hydroxy-3-(9-oxo-9H-thioxanthen-4-yloxy)-N,N,N-trimethyl-1-propaneaminium chloride, 2-hydroxy-3-(1-methyl-9-oxo-9H-thioxanthen-4-yloxy)-N,N,N-trimethyl-1-propaneaminium chloride, 2-hydroxy-3-(9-oxo-9H-thioxanthen-2-yloxy)-N,N,N-trimethyl-1-propaneaminium chloride, 2-hydroxy-3-(3,4-dimethyl-9-oxo-9H-thioxanthen-2-yloxy)-N,N,N-trimethyl-1-propaneaminium chloride, 2-hydroxy-3-(3,4-dimethyl-9H-thioxanthen-2-yloxy)-N,N,N-trimethyl-1-propaneaminium chloride, and 2-hydroxy-3-(1,3,4-trimethyl-9-oxo-9H-thioxanthen-2-yloxy)-N,N,N-trimethyl-1-propaneaminium chloride.

Examples of the water-soluble acylphosphine oxides include acylphosphine oxides represented by the following general formula (2) or (3).

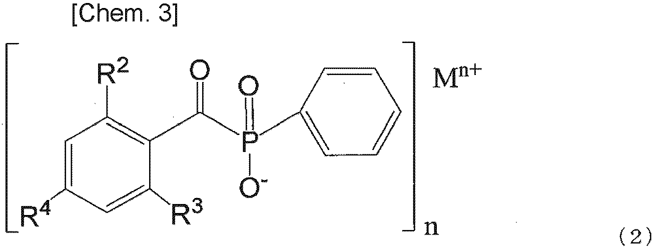

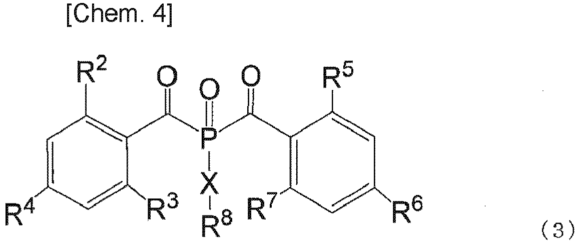

In formulae (2) and (3), R², R³, R⁴, R⁵, R⁶, and R⁷ are each independently a C₁ to C₄ linear or branched alkyl group or a halogen atom, M is a hydrogen ion, an alkali metal ion, an alkali earth metal ion, a magnesium ion, a pyridinium ion (the pyridine ring may have a substituent), or an ammonium ion represented by HN⁺R⁹R¹⁰R¹¹ (where R⁹, R¹⁰, and R¹¹ are each independently an organic group or a hydrogen atom), n is 1 or 2, X is a C₁ to C₄ linear or branched alkylene group, and R⁸ represents - CH(CH₃)COO(C₂H₄O)ₚCH₃, where p represents an integer of 1 to 1,000.

The alkyl group represented by R², R³, R⁴, R⁵, R⁶, and R⁷ is not particularly limited, as long as it is a C₁ to C₄ linear or branched alkyl group. Examples include methyl, ethyl, n-propyl, isopropyl, n-butyl, isobutyl, sec-butyl, 2-methylpropyl, and tert-butyl. The alkyl group represented by R², R³, R⁴, R⁵, R⁶, and R⁷ is preferably a C₁ to C₃ linear alkyl group, more preferably methyl or ethyl, even more preferably methyl. Examples of X include methylene, ethylene, n-propylene, isopropylene, and n-butylene. X is preferably a C₁ to C₃ linear alkylene group, more preferably methylene or ethylene, even more preferably methylene.

Examples of the substituent of the pyridine ring when M is a pyridinium ion include halogen atoms (a fluorine atom, a chlorine atom, a bromine atom, an iodine atom), a carboxyl group, a C₂ to C₆ linear or branched acyl group, a C₁ to C₆ linear or branched alkyl group, and a C₁ to C₆ linear or branched alkoxy group. Preferably, M is an alkali metal ion, an alkali earth metal ion, a magnesium ion, a pyridinium ion (the pyridine ring may have a substituent), or an ammonium ion represented by HN⁺R⁹R¹⁰R¹¹ (the symbols have the same meaning as above). Examples of the alkali metal ion include a lithium ion, a sodium ion, a potassium ion, a rubidium ion, and a cesium ion. Examples of the alkali earth metal ion include a calcium ion, a strontium ion, a barium ion, and a radium ion. The organic group represented by R⁹, R¹⁰, and R¹¹ may be the same group exemplified above for the substituent of the pyridine ring (excluding a halogen atom).

In view of storage stability and shade stability in a composition of the self-adhesive dental composite resin (X), particularly preferred are compounds in which R², R³, R⁴, R⁵, R⁶, and R⁷ are all methyl groups. Examples of ammonium ions include ammonium ions derived from various amines. Examples of the amines include ammonia, trimethylamine, diethylamine, dimethylaniline, ethylenediamine, triethanolamine, N,N-dimethylaminomethacrylate, 4-(N,N-dimethylamino)benzoic acid and alkyl esters thereof, 4-(N,N-diethylamino)benzoic acid and alkyl esters thereof, and N,N-bis(2-hydroxyethyl)-p-toluidine.

In view of adhesive properties, p in R⁸ is preferably 1 or more, more preferably 2 or more, even more preferably 3 or more, particularly preferably 4 or more, and is preferably 1,000 or less, more preferably 100 or less, even more preferably 75 or less, particularly preferably 50 or less.

Particularly preferred among these water-soluble acylphosphine oxides are sodium phenyl(2,4,6-trimethylbenzoyl)phosphinate, lithium phenyl(2,4,6-trimethylbenzoyl)phosphinate, and compounds represented by general formula (3) and in which the moiety corresponding to the group represented by R⁸ is synthesized from polyethylene glycol methyl ether methacrylate having a molecular weight of 950.

The water-soluble acylphosphine oxides having such structures can be synthesized according to known methods, and some are available as commercially available products. For example, the methods disclosed in JP S57-197289 A and WO2014/095724 can be used for the synthesis of the water-soluble acylphosphine oxides. The water-soluble photopolymerization initiator (c-1a) may be used alone, or two or more thereof may be used in combination.

The water-soluble photopolymerization initiator (c-1a) may be dissolved in the self-adhesive dental composite resin (X), or may be dispersed in the form of a powder in a composition of the self-adhesive dental composite resin (X).

When the water-soluble photopolymerization initiator (c-1a) is dispersed in the form of a powder in a composition of the self-adhesive dental composite resin (X), the average particle diameter is preferably 500 µm or less, more preferably 100 µm or less, even more preferably 50 µm or less because the water-soluble photopolymerization initiator (c-1a) tends to settle when the average particle diameter is excessively large. The average particle diameter is preferably 0.01 µm or more because the specific surface area of the powder overly increases, and the amount of powder that can be dispersed in a composition of the self-adhesive dental composite resin (X) decreases when the average particle diameter is excessively small. Taken together, the average particle diameter of water-soluble photopolymerization initiator (c-1a) preferably ranges from 0.01 to 500 µm, more preferably 0.01 to 100 µm, even more preferably 0.01 to 50 µm.

The average particle diameter of a powder of individual water-soluble photopolymerization initiators (c-1a) can be determined by taking an electron micrograph of at least 100 particles, and calculating the volume average particle diameter from the captured image after an image analysis with image-analyzing particle size distribution measurement software (Mac-View, manufactured by Mountech Co., Ltd.).

The shape of the water-soluble photopolymerization initiator (c-1a) when it is dispersed in the form of a powder is not particularly limited, and may be any of various shapes, including, for example, spherical, stylus, plate-like, and crushed shapes. The water-soluble photopolymerization initiator (c-1a) can be prepared using a known method such as pulverization, freeze drying, or reprecipitation. In view of the average particle diameter of the powder obtained, freeze drying and reprecipitation are preferred, and freeze drying is more preferred.

In view of curability and other properties of the self-adhesive dental composite resin (X) obtained, the content of water-soluble photopolymerization initiator (c-1a) is preferably 0.01 to 20 parts by mass relative to total 100 parts by mass of monomers in a self-adhesive dental composite resin (X) of the present invention. In view of adhesive properties to tooth structure, the content of water-soluble photopolymerization initiator (c-1a) is more preferably 0.05 to 10 parts by mass, even more preferably 0.1 to 5 parts by mass relative to total 100 parts by mass of monomers in a self-adhesive dental composite resin (X) of the present invention. When the content of water-soluble photopolymerization initiator (c-1a) is at or above these lower limits, polymerization can sufficiently proceed at the bonding interface, making it easier to provide a sufficient bond strength. When the content of water-soluble photopolymerization initiator (c-1a) is at or below the foregoing upper limits, it is easier to provide a sufficient bond strength.

### · Water-Insoluble Photopolymerization Initiator (c-1b)

In view of curability, a self-adhesive dental composite resin (X) of the present invention preferably comprises a water-insoluble photopolymerization initiator (c-1b) having a solubility of less than 10 g/L in 25°C water (hereinafter, also referred to as "water-insoluble photopolymerization initiator (c-1b)"), in addition to the water-soluble photopolymerization initiator (c-1a). The water-insoluble photopolymerization initiator (c-1b) used in the present invention may be a known photopolymerization initiator. The water-insoluble photopolymerization initiator (c-1b) may be contained alone, or two or more thereof may be contained in combination.

Examples of the water-insoluble photopolymerization initiator (c-1b) include (bis)acylphosphine oxides (other than those exemplified for the water-soluble photopolymerization initiator (c-1a)), thioxanthones, ketals, α-diketones, coumarins, anthraquinones, benzoin alkyl ether compounds, and α-aminoketone compounds.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, and benzoyl di(2,6-dimethylphenyl)phosphonate. Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,5,6-trimethylbenzoyl)-2,4,4-trimethylpentylphosphine oxide.

Examples of the thioxanthones include thioxanthone, and 2-chlorothioxanthen-9-one.

Examples of the ketals include benzyl dimethyl ketal, and benzyl diethyl ketal.

Examples of the α-diketones include diacetyl, benzyl, dl-camphorquinone, 2,3-pentadione, 2,3-octadione, 9,10-phenanthrenequinone, 4,4'-oxybenzyl, and acenaphthenequinone. Particularly preferred is dl-camphorquinone for its maximum absorption wavelength occurring in the visible light region.

Examples of the coumarins include compounds mentioned in JP H09-3109 A and JP H10-245525A, including, for example, 3,3'-carbonylbis(7-diethylaminocoumarin), 3-(4-methoxybenzoyl)coumarin, 3-thienoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzoimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetramethyl-1H,5H,11H-[1]benzopyrrano[6,7,8-ij]quinolizin-11-one, and 10-(2-benzothiazolyl)-2,3,6,7-tetrahydro-1,1,7,7-tetramethyl-1H,5H,11H-[1]benzopyrrano[6,7,8-ij]quinolizin-11-one.

Particularly preferred among these coumarins are 3,3'-carbonylbis(7-diethylaminocoumarin) and 3,3'-carbonylbis(7-dibutylaminocoumarin).

Examples of the anthraquinones include anthraquinone, 1-chloroanthraquinone, 2-chloroanthraquinone, 1-bromoanthraquinone, 1,2-benzanthraquinone, 1-methylanthraquinone, 2-ethylanthraquinone, and 1-hydroxyanthraquinone.

Example of the benzoin alkyl ether compounds include benzoin methyl ether, benzoin ethyl ether, benzoin isopropyl ether, and benzoin isobutyl ether.

Examples of the α-aminoketone compounds include 2-methyl-1-[4-(methylthio)phenyl]-2-morpholinopropan-1-one.

Among these examples, the water-insoluble photopolymerization initiator (c-1b) is preferably at least one selected from the group consisting of a (bis)acylphosphine oxide, an α-diketone, and a coumarin. In this way, a self-adhesive dental composite resin (X) can be obtained that has excellent photocurability both in the visible light region and the near ultraviolet region, and that shows sufficient photocurability regardless of whether the light source used is a halogen lamp, a light emitting diode (LED), or a xenon lamp.

The content of water-insoluble photopolymerization initiator (c-1b) is not particularly limited. However, in view of curability and other properties of the composition of self-adhesive dental composite resin (X) obtained, the content of water-insoluble photopolymerization initiator (c-1b) is preferably 0.01 to 10 parts by mass, more preferably 0.05 to 7 parts by mass, even more preferably 0.1 to 5 parts by mass relative to total 100 parts by mass of monomers in a self-adhesive dental composite resin (X) of the present invention. When the content of water-insoluble photopolymerization initiator (c-1b) is at or below these upper limits, it is easier to obtain a sufficient bond strength even when the water-insoluble photopolymerization initiator (c-1b) itself has low polymerization performance, in addition to reducing a possibility of precipitation of the water-insoluble photopolymerization initiator (c-1b) itself from the self-adhesive dental composite resin (X).

When the water-soluble photopolymerization initiator (c-1a) and the water-insoluble photopolymerization initiator (c-1b) are used in combination, the mass ratio (c-1a): (c-1b) of water-soluble photopolymerization initiator (c-1a) and water-insoluble photopolymerization initiator (c-1b) in the present invention is preferably 10:1 to 1:10, more preferably 7:1 to 1:7, even more preferably 5:1 to 1:5, particularly preferably 3:1 to 1:3. When the fraction of water-soluble photopolymerization initiator (c-1a) in the mass ratio exceeds 10:1, the curability of the self-adhesive dental composite resin (X) itself decreases, and the composition may have difficulty in exhibiting high bond strength. When the fraction of water-insoluble photopolymerization initiator (c-1b) in the mass ratio exceeds 1:10, the self-adhesive dental composite resin (X) may have difficulty in exhibiting high bond strength as a result of insufficient promotion of polymerization at the bonding interface, though the curability of the composition itself can increase.

### . Chemical Polymerization Initiator (c-2)

A self-adhesive dental composite resin (X) of the present invention may additionally comprise a chemical polymerization initiator (c-2). Preferred for use as chemical polymerization initiator (c-2) are organic peroxides. The organic peroxide used as chemical polymerization initiator (c-2) is not particularly limited, and known organic peroxides may be used. Typical examples of such organic peroxides include ketone peroxides, hydroperoxides, diacylperoxides, dialkylperoxides, peroxyketals, peroxyesters, and peroxydicarbonates. Specific examples of these organic peroxides include those mentioned in WO2008/087977. The chemical polymerization initiator (c-2) may be used alone, or two or more thereof may be used in combination.

### <Filler (d)>

It is preferable that a self-adhesive dental composite resin (X) of the present invention comprise a filler (d), in order to adjust the ease of handling, and to increase the mechanical strength of the cured product. Examples of such fillers include inorganic fillers, organic-inorganic composite fillers, and organic fillers. The filler (d) may be used alone, or two or more thereof may be used in combination.

Examples of the materials of the inorganic fillers include quartz, silica, alumina, silica-titania, silica-titania-barium oxide, silica-zirconia, silica-alumina, lanthanum glass, borosilicate glass, soda glass, barium glass, strontium glass, glass-ceramics, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, strontium calcium fluoroaluminosilicate glass, ytterbium oxide, and silica-coated ytterbium fluoride. These may be used alone, or two or more thereof may be used as a mixture. The shape of inorganic filler is not particularly limited, and the particle diameter of filler may be appropriately selected for use. For considerations such as superiority of the mechanical strength and transparency of the self-adhesive dental composite resin (X) obtained, preferred for use are quartz, silica, silica-zirconia, barium glass, ytterbium oxide, and silica-coated ytterbium fluoride, more preferably quartz, silica, silica-zirconia, barium glass, and silica-coated ytterbium fluoride. In view of considerations such as the ease of handling and mechanical strength of the self-adhesive dental composite resin (X) obtained, the average particle diameter of the inorganic filler is preferably 0.001 to 50 µm, more preferably 0.001 to 10 µm. A certain preferred embodiment is, for example, a self-adhesive dental composite resin (X) in which the filler (d) is an inorganic filler. In the present invention, the average particle diameter of inorganic filler means the average particle diameter before surface treatment when the inorganic filler is subjected to a surface treatment, as will be described.

The inorganic filler may be, for example, an irregularly shaped filler or a spherical filler. In view of improving the mechanical strength of a cured product of the self-adhesive dental composite resin (X), the inorganic filler used is preferably a spherical filler. Here, the spherical filler used in the present invention represents particles that appear round in shape when observed in a unit field of an electron micrograph, and that have an average uniformity of 0.6 or more as calculated by dividing the diameter of a particle perpendicular to its maximum diameter by the maximum diameter. The spherical filler has an average particle diameter of preferably 0.05 to 5 µm. When the average particle diameter is less than 0.05 µm, the mechanical strength may decrease as a result of a decrease of the filling rate of the spherical filler in the self-adhesive dental composite resin (X). When the average particle diameter is more than 5 µm, the spherical filler has a reduced surface area, and the self-adhesive dental composite resin (X) may fail to produce a cured product having high mechanical strength.

In order to adjust the flowability of the self-adhesive dental composite resin (X), the inorganic filler may be used after an optional surface treatment with a known surface treatment agent such as a silane coupling agent. Examples of the surface treatment agent include vinyltrimethoxysilane, vinyltriethoxysilane, vinyltrichlorosilane, vinyl tri(β-methoxyethoxy)silane, γ-methacryloyloxypropyltrimethoxysilane, 8-methacryloyloxyoctyltrimethoxysilane, 11-methacryloyloxyundecyltrimethoxysilane, γ-glycidoxypropyltrimethoxysilane, γ-mercaptopropyltrimethoxysilane, and γ-aminopropyltriethoxysilane.

The surface treatment can be carried out using any known method, including, for example, a method that adds the surface treatment agent by spraying it while vigorously stirring the inorganic filler, a method that disperses or dissolves the inorganic filler and surface treatment agent in a suitable solvent, and removes the solvent, and a method in which the alkoxy group of the surface treatment agent is transformed into a silanol group by hydrolysis with an acid catalyst in an aqueous solution, and the silanol group is allowed to adhere to the inorganic filler surface in the aqueous solution before removal of water. In all of these methods, the reaction between the inorganic filler surface and the surface treatment agent can proceed to completion to enable a surface treatment by applying heat in a temperature range of typically 50 to 150°C. The amount of surface treatment agent is not particularly limited, and the surface treatment agent may be used in an amount of, for example, 1 to 10 parts by mass relative to 100 parts by mass of the inorganic filler before treatment.

The organic-inorganic composite filler used in the present invention is a filler obtained by adding a monomer to the inorganic filler, polymerizing the mixture in paste form, and pulverizing the polymerized filler. The organic-inorganic composite filler may be, for example, a TMPT filler (a filler obtained by mixing trimethylolpropane methacrylate and a silica filler, and pulverizing the mixture after polymerization). The shape of the organic-inorganic composite filler is not particularly limited, and the particle diameter of filler may be appropriately selected for use. In view of properties such as the ease of handling and mechanical strength of the composition of self-adhesive dental composite resin (X) obtained, the organic-inorganic composite filler has an average particle diameter of preferably 0.001 to 50 µm, more preferably 0.001 to 10 µm.

Examples of the materials of the organic fillers include polymethylmethacrylate, polyethylmethacrylate, a methyl methacrylate-ethyl methacrylate copolymer, crosslinked polymethylmethacrylate, crosslinked polyethylmethacrylate, polyamides, polyvinyl chloride, polystyrene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. These may be used alone, or two or more thereof may be used as a mixture. The shape of organic filler is not particularly limited, and the particle diameter of filler may be appropriately selected for use. In view of considerations such as the ease of handling and mechanical strength of the self-adhesive dental composite resin (X) obtained, the average particle diameter of the organic filler is preferably 0.001 to 50 µm, more preferably 0.001 to 10 µm.

In this specification, the average particle diameter of filler can be determined by a laser diffraction scattering method or by observing particles with an electron microscope. Specifically, a laser diffraction scattering method is more convenient for the measurement of particles having a particle size of 0.1 µm or more, whereas electron microscopy is a more convenient method of particle size measurement for ultrafine particles of less than 0.1 µm. Here, 0.1 µm is a measured value by a laser diffraction scattering method.

As a specific example of a laser diffraction scattering method, the particle size may be measured by volume using, for example, a laser diffraction particle size distribution analyzer (SALD-2300, manufactured by Shimadzu Corporation) with a 0.2% sodium hexametaphosphate aqueous solution used as dispersion medium.

In electron microscopy, for example, particles may be photographed with an electron microscope (Model S-4000, manufactured by Hitachi), and the size of particles (at least 200 particles) observed in a unit field of the captured image may be measured using image-analyzing particle-size-distribution measurement software (Mac-View, manufactured by Mountech Co., Ltd.). Here, the particle diameter is determined as an arithmetic mean value of the maximum and minimum lengths of particles, and the average primary particle diameter is calculated from the number of particles and the particle diameter.

In a self-adhesive dental composite resin (X) of the present invention, it is preferable to use a mixture or a combination of two or more fillers of different materials, different particle size distributions, and different forms. By combining two or more types of fillers, the fillers can interact with the monomer or with the other fillers at an increased number of points, in addition to densely packing themselves. Depending on the type of filler, it is also possible to control paste fluidity in the presence or absence of a shear force. In view of the ease of handling and paste properties of a self-adhesive dental composite resin (X) of the present invention, the filler (d) is preferably a combination (I) of a filler (d-1) having an average particle diameter of 1 nm or more and less than 0.1 µm, and a filler (d-2) having an average particle diameter of 0.1 µm or more and 1 µm or less; a combination (II) of a filler (d-1) having an average particle diameter of 1 nm or more and less than 0.1 µm, and a filler (d-3) having an average particle diameter of more than 1 µm and 10 µm or less; a combination (III) of a filler (d-1) having an average particle diameter of 1 nm or more and less than 0.1 µm, a filler (d-2) having an average particle diameter of 0.1 µm or more and 1 µm or less, and a filler (d-3) having an average particle diameter of more than 1 µm and 10 µm or less; or a combination (IV) of fillers (d-2) having an average particle diameter of 0.1 µm or more and 1 µm or less. In view of paste properties and cavity sealing properties, the combinations (I), (II), and (III) are more preferred, and the combinations (I) and (II) are even more preferred. The combination (IV) of fillers (d-2) having an average particle diameter of 0.1 µm or more and 1 µm or less means an embodiment that comprises two types of fillers (d-2) of different average particle diameters between 0.1 µm and 1 µm. The fillers (d) of different particle diameters may include other types of fillers, provided that the fillers (d) have the foregoing combinations. Unintended inclusion of non-filler particles as impurities is acceptable to such an extent that it does not hinder the effectiveness of the present invention.

The content of filler (d) is not particularly limited. In view of the mechanical strength of the cured product and adhesive properties to tooth structure, the content of filler (d) is preferably 50 to 90 parts by mass, more preferably 55 to 85 parts by mass, even more preferably 60 to 80 parts by mass in total 100 parts by mass of self-adhesive dental composite resin (X).

A method of production of self-adhesive dental composite resin (X) is not particularly limited, as long as it produces a self-adhesive dental composite resin (X) comprising a monomer (a) having an acidic group, a monomer (b) having no acidic group, a polymerization initiator (c), and a filler (d). A self-adhesive dental composite resin (X) of the present invention can be produced with ease using a method known to a person skilled in the art.

### <Polymerization Accelerator (e)>

A self-adhesive dental composite resin (X) may comprise a polymerization accelerator (e), in addition to the water-soluble photopolymerization initiator (c-1a), water-insoluble photopolymerization initiator (c-1b), and chemical polymerization initiator (c-2). Examples of the polymerization accelerator (e) that can be used in the present invention include amines, sulfinic acid and salts thereof, borate compounds, barbituric acid compounds, triazine compounds, copper compounds, tin compounds, vanadium compounds, halogen compounds, aldehydes, thiol compounds, sulfites, bisulfites, and thiourea compounds.

The amines used as polymerization accelerator (e) can be categorized into aliphatic amines and aromatic amines. Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In view of the curability and storage stability of the self-adhesive dental composite resin (X), preferred for use are tertiary aliphatic amines, more preferably N-methyldiethanolamine and triethanolamine.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-diisopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, propyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of the ability to impart excellent curability to the self-adhesive dental composite resin (X), it is preferable to use at least one selected from the group consisting of N,N-bis(2-hydroxyethyl)-p-toluidine, ethyl 4-(N,N-dimethylamino)benzoate, n-butoxyethyl 4-(N,N-dimethylamino)benzoate, and 4-(N,N-dimethylamino)benzophenone.

Specific examples of sulfinic acid and salts thereof, borate compounds, barbituric acid compounds, triazine compounds, copper compounds, tin compounds, vanadium compounds, halogen compounds, aldehydes, thiol compounds, sulfites, bisulfites, and thiourea compounds include those mentioned in WO2008/087977.

The polymerization accelerator (e) may be contained alone, or two or more thereof may be contained in combination. The content of the polymerization accelerator (e) used in the present invention is not particularly limited. However, in view of the curability and other properties of the self-adhesive dental composite resin (X) obtained, the content of polymerization accelerator (e) is preferably 0.001 to 30 parts by mass, more preferably 0.01 to 10 parts by mass, particularly preferably 0.1 to 5 parts by mass relative to total 100 parts by mass of monomers in the self-adhesive dental composite resin (X). When the content of polymerization accelerator (e) is at or above these lower limits, it is easier to provide a sufficient bond strength by allowing polymerization to sufficiently proceed.

In this respect, the content of polymerization accelerator (e) is more preferably 0.05 parts or more by mass. When the content of polymerization accelerator (e) is at or below the foregoing upper limits, it is easier to provide sufficient adhesive properties, and to reduce precipitation of the polymerization accelerator (e) itself from the self-adhesive dental composite resin (X). In this respect, the content of polymerization accelerator (e) is more preferably 20 parts or less by mass.

### <Fluorine-Ion Releasing Substance>

The self-adhesive dental composite resin (X) may additionally comprise a fluorine-ion releasing substance. By containing a fluorine-ion releasing substance, the self-adhesive dental composite resin (X) produced can impart acid resistance to tooth structure. Examples of the fluorine-ion releasing substance include metal fluorides such as sodium fluoride, potassium fluoride, sodium monofluorophosphate, lithium fluoride, and ytterbium fluoride. The fluorine-ion releasing substance may be contained alone, or two or more thereof may be contained in combination.

The self-adhesive dental composite resin (X) may also comprise a known additive, provided that such additives do not cause a performance drop. Examples of additives that may be contained in the self-adhesive dental composite resin (X) include polymerization inhibitors, antioxidants, pigments, dyes, ultraviolet absorbers, solvents such as organic solvents, and thickeners. The additives may be used alone, or two or more thereof may be used in combination. In certain embodiments, the content of the solvent (for example, water, organic solvent) in the self-adhesive dental composite resin (X) is preferably less than 1 mass%, more preferably less than 0.1 mass%, even more preferably less than 0.01 mass% based on the total mass of the self-adhesive dental composite resin (X).

Examples of the polymerization inhibitors include hydroquinone, hydroquinone monomethyl ether, dibutylhydroquinone, dibutylhydroquinone monomethyl ether, t-butylcatechol, 2-t-butyl-4,6-dimethylphenol, 2,6-di-t-butylphenol, and 3,5-di-t-butyl-4-hydroxytoluene. The content of the polymerization inhibitor is preferably 0.001 to 1.0 parts by mass relative to total 100 parts by mass of monomers in the self-adhesive dental composite resin (X).

Examples of the fractions of the components in the self-adhesive dental composite resin (X) are as follows. By taking the total amount of monomers as 100 parts by mass, it is preferable to comprise 1 to 40 parts by mass of monomer (a) having an acidic group, and 60 to 99 parts by mass of monomer (b) having no acidic group in 100 parts by mass of the monomer components, and 0.05 to 10 parts by mass of photopolymerization initiator (c-1), 100 to 900 parts by mass of filler (d), and 0.001 to 30 parts by mass of polymerization accelerator (e) relative to total 100 parts by mass of the monomers. More preferably, it is preferable to comprise 2.5 to 35 parts by mass of monomer (a) having an acidic group, and 65 to 97.5 parts by mass of monomer (b) having no acidic group in total 100 parts by mass of the monomers, and 0.1 to 5 parts by mass of photopolymerization initiator (c-1), 120 to 560 parts by mass of filler (d), and 0.01 to 10 parts by mass of polymerization accelerator (e) relative to total 100 parts by mass of the monomers. Even more preferably, it is preferable to comprise 5 to 30 parts by mass of monomer (a) having an acidic group, and 70 to 95 parts by mass of monomer (b) having no acidic group in total 100 parts by mass of the monomers, and 0.15 to 2.5 parts by mass of photopolymerization initiator (c-1), 150 to 400 parts by mass of filler (d), and 0.1 to 5 parts by mass of polymerization accelerator (e) relative to total 100 parts by mass of the monomers.

The following describes the components used in a dental cleaning material (Y) of the present invention.

The dental cleaning material (Y) is a material used for applications such as cavities, abutment teeth, and dental restorations. It is used to clean the applied surfaces by removing contamination, or protein stains from saliva and blood on these surfaces. The material can be used for purposes beyond cleaning of contaminated surfaces.

The dental cleaning material (Y) is intended for cleaning structures such as cavities, abutment teeth, and dental restorations, and the components contained in the cleaning material should preferably be removed. As such, a method of its use must include the process of removing contaminants and the components of the dental cleaning material (Y) from the applied surfaces by wiping or washing with water to such an extent that it does not hinder the effectiveness of the present invention. For this reason, it is preferable that the method do not include the process of curing the dental cleaning material (Y) itself.

It is important that the dental cleaning material (Y) have a pH of 2.0 or more and less than 9.0. With this pH range, the dental cleaning material (Y) does not demineralize the tooth structure while enabling removal of the smear layer and contamination due to protein-containing bodily fluids, and does not alter the state of the tooth structure on surfaces bonded to the self-adhesive dental composite resin (X). If the removal of the smear layer is insufficient, the presence of the smear layer may lead to a decrease of the bond strength because the smear layer itself is low in mechanical strength.

One of the characteristics of the self-adhesive composite resin (X) is that it shows even superior bond strength to bonding surfaces when the tooth structure is maintaining its state without undergoing demineralization on these surfaces. The self-adhesive dental composite resin (X) is therefore able to increase its bond strength to tooth structure when applied to the tooth structure after cleaning with the dental cleaning material (Y).

### <Cationic Component (f)>

The dental cleaning material (Y) preferably comprises a cationic component (f). The cationic component (f) reacts with an anionic component (g) (described later) to form salts, and improves the solubility in water, allowing more efficient removal of only the smear layer, without demineralizing the tooth structure. The tooth structure is therefore able to maintain its state on surfaces bonded to the self-adhesive dental composite resin (X). The cationic component (f) also serves as a material that adjusts the pH of the dental cleaning material (Y). The cationic component (f) is not particularly limited, and is preferably a basic compound. The basic compound is preferably an organic amine compound (f-1), a basic inorganic compound (f-2), or an antimicrobial salt compound (f-3). In view of solubility in water and the storage stability of the dental cleaning material (Y), the basic compound is more preferably an organic amine compound (f-1). The cationic component (f) may be contained alone, or two or more thereof may be used in combination.

### . Organic Amine Compound (f-1)

The organic amine compound (f-1) improves the cleaning effect of dental cleaning material (Y) on contamination due to protein-containing bodily fluids or substances derived from protein-containing bodily fluids, and enhances the solubility of cationic component (f) in water. Because of its consistent performance, the organic amine compound (f-1) can improve a dental filling kit of the present invention in terms of a bond strength to tooth structure and cavity sealing properties.

Additionally, some of the organic amine compound (f-1) react with an anionic component (g) to form salts. With its surfactant action, the organic amine compound (f-1) can further enhance the cleaning effect on the smear layer and contamination due to protein-containing bodily fluids. By containing the organic amine compound (f-1), the dental cleaning material (Y) can more efficiently remove the smear layer and contamination due to protein-containing bodily fluids, without demineralizing the tooth structure. The tooth structure is therefore able to maintain its state on surfaces bonded to the self-adhesive dental composite resin (X), increasing the bond strength to tooth structure after cleaning with the dental cleaning material (Y), and more greatly improving the bond strength to tooth structure and the cavity sealing properties. The organic amine compound (f-1) may comprise a primary organic amine compound or a secondary organic amine compound. However, in view of inhibiting side reactions, it is preferable that the organic amine compound (f-1) comprise a tertiary organic amine compound. The organic amine compound (f-1) may be an aliphatic compound or an aromatic compound. In view of the ability to impart an even superior bond strength and even superior cavity sealing properties to dental cleaning material (Y) on tooth structure, it is preferable in certain embodiments that the organic amine compound (f-1) comprise a tertiary organic amine compound as an aliphatic compound having one or more hydroxyl groups, or a tertiary organic amine compound as an aromatic compound having one or more hydroxyl groups, more preferably a tertiary organic amine compound as an aliphatic compound having two or more hydroxyl groups, or a tertiary organic amine compound as an aromatic compound having two or more hydroxyl groups.

Examples of the aliphatic tertiary organic amine compounds include N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In terms of improving the bond strength to tooth structure and the cavity sealing properties, more preferred are triethanolamine, and 2-(dimethylamino)ethyl methacrylate.

Examples of the aromatic tertiary organic amine compounds include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, methyl 4-(N,N-dimethylamino)benzoate, ethyl 4-(N,N-dimethylamino)benzoate, butyl 4-(N,N-dimethylamino)benzoate, n-butoxyethyl 4-(N,N-dimethylamino)benzoate, 2-(methacryloyloxy)ethyl 4-(N,N-dimethylamino)benzoate, and 4-(N,N-dimethylamino)benzophenone. More preferred is N,N-bis(2-hydroxyethyl)-p-toluidine for its consistent performance, and the ability to improve the bond strength to tooth structure and the cavity sealing properties by increasing the bond strength to tooth structure after cleaning with the dental cleaning material (Y).

The organic amine compound (f-1) may be contained alone, or two or more thereof may be contained in combination. The content of organic amine compound (f-1) is not particularly limited, as long as the present invention can exhibit its effects. However, in view of the pH of dental cleaning material (Y) (a solution or a suspension), the content of organic amine compound (f-1) ranges preferably from 0.1 to 20 parts by mass, more preferably from 0.2 to 10 parts by mass, even more preferably from 0.25 to 5 parts by mass in total 100 parts by mass of the dental cleaning material (Y). When the content of organic amine compound (f-1) is at or above these lower limits, it is easier to produce the effect of adding organic amine compound (f-1). When the content of organic amine compound (f-1) is at or below the foregoing upper limits, it is possible to provide superior oral safety by inhibiting protein denaturation due to excessive basicity.

### . Basic Inorganic Compound (f-2)

A certain preferred embodiment is, for example, a dental cleaning material (Y) comprising a basic inorganic compound (f-2). The basic inorganic compound (f-2) is preferably at least one basic substance selected from a phosphate, hydrogen phosphate, and dihydrogen phosphate. These compounds, even with small amounts of addition, enhance the cleaning effect on the smear layer and contamination due to protein-containing bodily fluids on the tooth structure. Some of the basic inorganic compound (f-2) react with the anionic component (g) to form salts. With its surfactant action, the basic inorganic compound (f-2) can enhance the cleaning effect on the smear layer and contamination due to protein-containing bodily fluids. By containing the basic inorganic compound (f-2) in the dental cleaning material (Y), it is possible to increase the bond strength to tooth structure after cleaning with the dental cleaning material (Y), providing superior bond strength to tooth structure and superior cavity sealing properties after washing. Examples of the basic inorganic compound (f-2) include alkali metal salts of phosphoric acid, alkali metal salts of hydrogen phosphate, and alkali metal salts of dihydrogen phosphate, such as trisodium phosphate, disodium hydrogen phosphate, sodium dihydrogen phosphate, tripotassium phosphate, dipotassium hydrogen phosphate, and potassium dihydrogen phosphate; alkali-earth metal salts of phosphoric acid, alkali-earth metal salts of hydrogen phosphate, and alkali-earth metal salts of dihydrogen phosphate, such as tricalcium phosphate, calcium hydrogen phosphate, and calcium dihydrogen phosphate; and trimagnesium phosphate, and magnesium hydrogen phosphate. The basic inorganic compound (f-2) is preferably at least one selected from the group consisting of an alkali metal salt of phosphoric acid, an alkali metal salt of hydrogen phosphate, and an alkali metal salt of dihydrogen phosphate. More preferred are trisodium phosphate, disodium hydrogen phosphate, sodium dihydrogen phosphate, tripotassium phosphate, dipotassium hydrogen phosphate, and potassium dihydrogen phosphate.

The basic inorganic compound (f-2) may be contained alone, or two or more thereof may be contained in combination. The content of basic inorganic compound (f-2) is not particularly limited, as long as the present invention can exhibit its effects. In view of the pH of dental cleaning material (Y) (a solution or a suspension), the content of basic inorganic compound (f-2) ranges preferably from 0.1 to 20 parts by mass, more preferably from 0.2 to 10 parts by mass, even more preferably from 0.25 to 5 parts by mass in total 100 parts by mass of the dental cleaning material (Y). When the content of basic inorganic compound (f-2) is at or above these lower limits, it is easier to produce the effect of adding basic inorganic compound (f-2). With the content of basic inorganic compound (f-2) is at or below the foregoing upper limits, it is possible to provide superior oral safety by inhibiting protein denaturation due to excessive basicity. When the content of basic inorganic compound (f-2) is at or below the foregoing upper limits, it is possible to reduce precipitation of the basic inorganic compound (f-2) itself, providing superior ease of handling, and a sufficient cleaning effect on protein-containing bodily fluids.

### · Antimicrobial Salt Compound (f-3)

A certain preferred embodiment is, for example, a dental cleaning material (Y) comprising an antimicrobial salt compound (f-3). The antimicrobial salt compound (f-3) is not limited to particular compounds, provided that it is an organic salt compound that exhibits antimicrobial properties. The antimicrobial salt compound (f-3), even with small amounts of addition, shows antimicrobial effect, in addition to enhancing the cleaning effect on the smear layer and contamination due to protein-containing bodily fluids on the tooth structure. Some of the antimicrobial salt compound (f-3) react with an anionic component (g) to form salts. With its surfactant action, the antimicrobial salt compound (f-3) can enhance the cleaning effect on the smear layer and contamination due to protein-containing bodily fluids. By containing the antimicrobial salt compound (f-3) in the dental cleaning material (Y), it is possible to more efficiently remove the smear layer and contamination due to protein-containing bodily fluids, without demineralizing the tooth structure. The tooth structure is therefore able to maintain its state on bonding surfaces, providing superior bond strength to the tooth structure and superior cavity sealing properties after washing.

Examples of the antimicrobial salt compound (f-3) include trimethylhexadecylammonium chloride, triethyldodecylammonium bromide, benzalkonium chloride, benzethonium chloride, dimethyldidodecylammonium chloride, and various antimicrobial ammonium salt compounds represented by the following general formulae (4). 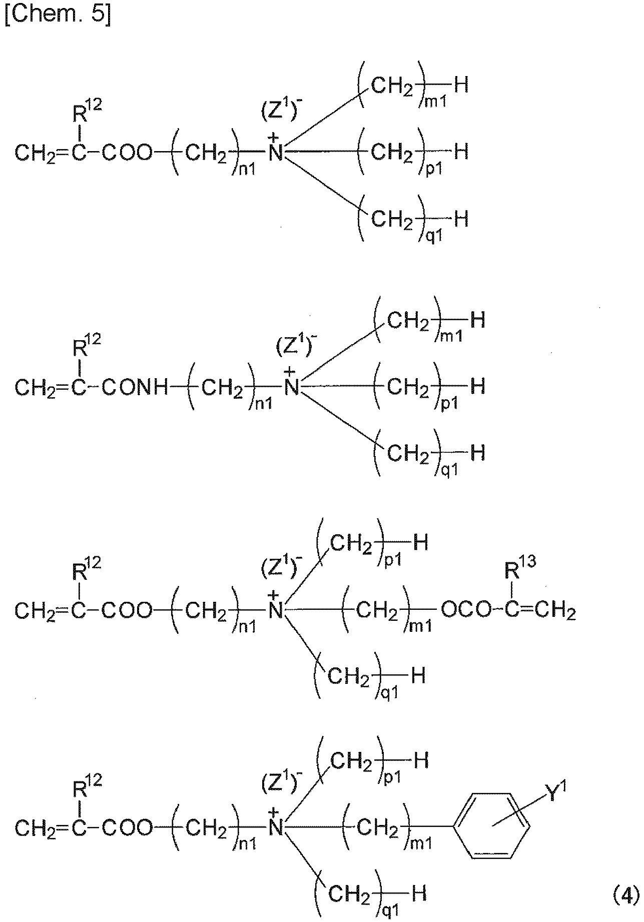 In formulae (4), R¹² and R¹³ each independently represent a hydrogen atom or a methyl group, Y¹ represents a methoxy group, an ethoxy group, an n-propoxy group, a chlorine atom, a bromine atom, a carboxy group, a hydroxyl group, a cyano group, or a vinyl group, (Z¹)⁻ represents a fluorine ion, a chlorine ion, a bromine ion, an iodine ion, (1/3PO₄)⁻, (1/2SO₄)⁻ CH₃SO₃⁻ or (CH₃COO)⁻, and the symbols n1, m1, p1, and q1 each independently represent an integer of 1 to 30.

Other examples include trimethylhexadecylphosphonium bromide, trimethyldodecylphosphonium chloride, dimethyldihexadecylphosphonium chloride, trimethylbenzylphosphonium chloride, and various antimicrobial phosphonium salt compounds represented by the following general formulae (5). 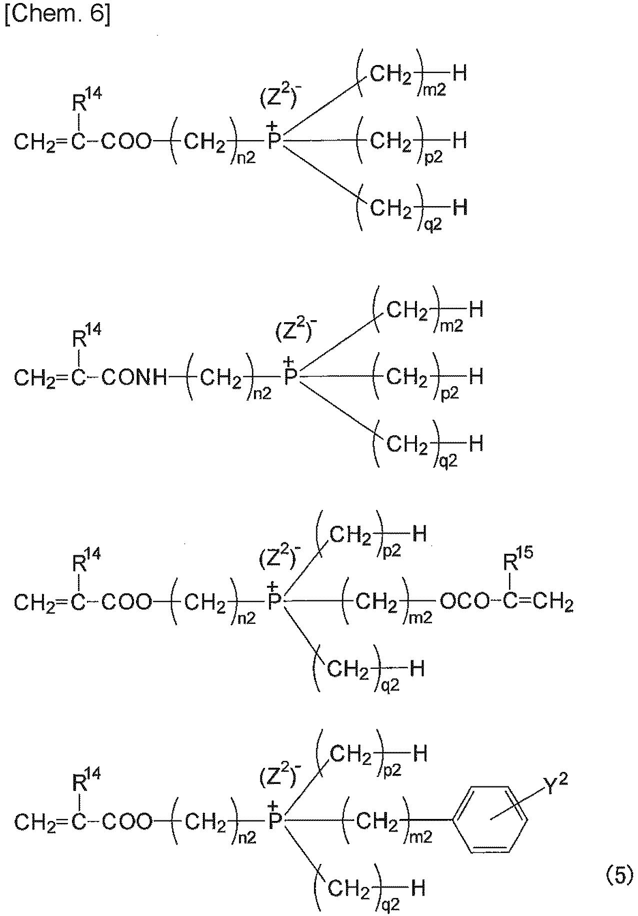 In formulae (5), R¹⁴ and R¹⁵ each independently represent a hydrogen atom or a methyl group, Y² represents a methoxy group, an ethoxy group, an n-propoxy group, a chlorine atom, a bromine atom, a carboxy group, a hydroxyl group, a cyano group, or a vinyl group, (Z²)⁻ represents a fluorine ion, a chlorine ion, a bromine ion, an iodine ion, (1/3PO₄)⁻, (1/2SO₄)⁻ CH₃SO₃⁻ or (CH₃COO)⁻, and the symbols n2, m2, p2, and q2 each independently represent an integer of 1 to 30.

Yet other examples include dodecylpyridinium chloride, dodecylpyridinium bromide, cetylpyridinium chloride, cetylpyridinium bromide, cetylpyridinium acetate, cetylpyridinium propionate, and various antimicrobial pyridinium salt compounds represented by the following general formulae (6). 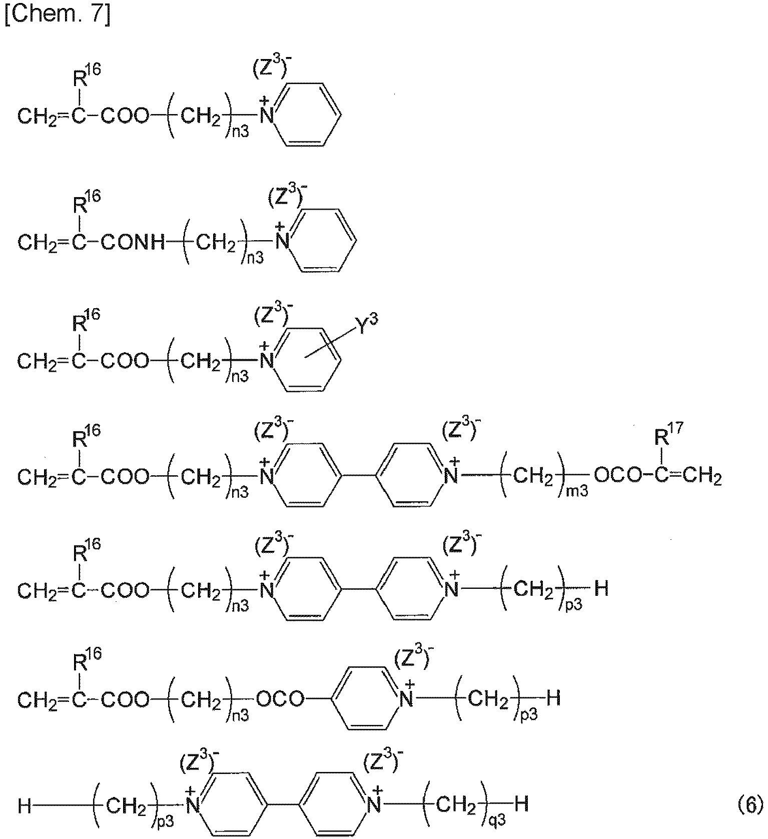 In formulae (6), R¹⁶ and R¹⁷ each independently represent a hydrogen atom or a methyl group, Y³ represents a methoxy group, an ethoxy group, an n-propoxy group, a chlorine atom, a bromine atom, a carboxy group, a hydroxyl group, or a cyano group, (Z³)⁻ represents a fluorine ion, a chlorine ion, a bromine ion, an iodine ion, (1/3PO₄)⁻, (1/2SO₄)⁻, CH₃SO₃⁻, or (CH₃COO)⁻, and the symbols n3, m3, p3, and q3 each independently represents an integer of 1 to 30.

In view of the cleaning effect on the smear layer and contamination due to protein-containing bodily fluids on the tooth structure, and concerning the antimicrobial effect as well, it is preferable that the antimicrobial salt compound (f-3) be a compound having a C10 to C20 carbon-chain alkyl group and alkylene group within the molecule. More preferred are antimicrobial pyridinium salt compounds for their ability to exhibit even superior antimicrobial properties. Also preferred in the present invention are antimicrobial salt compounds having a polymerizable group within the molecule. The antimicrobial salt compounds having a polymerizable group can be fixed through polymerization, and can effectively inhibit bacteria penetration through the bonding interface even after polymerization and cure. A dental cleaning material (Y) with even superior adhesive properties to tooth structure can be obtained when antimicrobial salt compounds having a polymerizable group are used, compared to when using antimicrobial salt compounds that do not contain a polymerizable group.

For advantages including ease of production, particularly preferred for use among the foregoing examples of antimicrobial salt compound (f-3) are methacrylic antimicrobial pyridinium salts represented by the following general formula (7), such as methacryloyloxydodecylpyridinium bromide (hereinafter, referred to with the abbreviation MDPB). 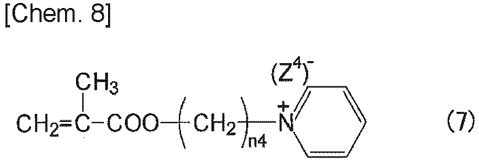 In formula (7), the symbol (Z⁴)⁻ represents a chlorine ion, a bromine ion, or an iodine ion, and n4 represents an integer of 12 to 25.

The method of synthesis of the methacrylic antimicrobial pyridinium salts is not particularly limited, and may be, for example, the method described in JP 2002-047118 A. Any known reagents can be used for synthesis without any restrictions. The methacrylic acid may be one that has been activated by halogenation, tosylation, or some other means.

The antimicrobial salt compound (f-3) may be contained alone, or two or more thereof may be contained in combination. The content of antimicrobial salt compound (f-3) is not particularly limited, as long as the present invention can exhibit its effects. However, in view of the pH of dental cleaning material (Y) (a solution or a suspension), the content of antimicrobial salt compound (f-3) ranges preferably from 0.1 to 20 parts by mass, more preferably from 0.2 to 10 parts by mass, even more preferably from 0.25 to 5 parts by mass in total 100 parts by mass of dental cleaning material (Y). When the content of antimicrobial salt compound (f-3) is at or above these lower limits, it is easier to produce the effect of adding antimicrobial salt compound (f-3). When the content of antimicrobial salt compound (f-3) is at or below the foregoing upper limits, it is possible to provide superior oral safety by inhibiting protein denaturation due to excessively strong antimicrobial properties. When the content of antimicrobial salt compound (f-3) is at or below the foregoing upper limits, it is possible to reduce precipitation of the antimicrobial salt compound (f-3) itself, providing superior ease of handling, and a sufficient cleaning effect on protein-containing bodily fluids.

In certain embodiments, the organic amine compound (f-1) and the basic inorganic compound (f-2) may be used in combination. The organic amine compound (f-1) and basic inorganic compound (f-2) have an organic amine compound (f-1):basic inorganic compound (f-2) mass ratio of preferably 10:0 to 1:1, more preferably 10:0 to 2:1, even more preferably 10:0 to 5:1.

In certain embodiments, the organic amine compound (f-1) and the antimicrobial salt compound (f-3) may be used in combination. The organic amine compound (f-1) and antimicrobial salt compound (f-3) have an organic amine compound (f-1):antimicrobial salt compound (f-3) mass ratio of preferably 10:0 to 1:1, more preferably 10:0 to 2:1, even more preferably 10:0 to 5:1.

In certain embodiments, the basic inorganic compound (f-2) and the antimicrobial salt compound (f-3) may be used in combination. The basic inorganic compound (f-2) and antimicrobial salt compound (f-3) have a basic inorganic compound (f-2):antimicrobial salt compound (f-3) mass ratio of preferably 10:0 to 1:1, more preferably 10:0 to 2:1, even more preferably 10:0 to 5:1.

### <Anionic Component (g)>

The dental cleaning material (Y) preferably comprises an anionic component (g). With its surfactant action, the anionic component (g) removes the smear layer and contamination due to protein-containing bodily fluids, and improves the cavity sealing properties. The anionic component (g) is not particularly limited, and may be, for example, a monomer (g-1) having an acidic group, or an organic compound (g-2) having no polymerizable group but having an acidic group. In view of improving adhesive properties to tooth structure, the anionic component (g) is preferably a monomer (g-1) having an acidic group.

### · Monomer (g-1) Having Acidic Group

The monomer (g-1) having an acidic group is a monomer having at least one acidic group such as a phosphoric acid group, a pyrophosphoric acid group, a thiophosphoric acid group, a phosphonic acid group, a carboxylic acid group, and a sulfonic acid group, and at least one radical polymerizable group such as an acryloyl group, a methacryloyl group, an acrylamide group, and a methacrylamide group. The monomer (g-1) having an acidic group is preferably at least one selected from the group consisting of a monomer having a phosphoric acid group, a monomer having a pyrophosphoric acid group, and a monomer having a carboxylic acid group. In view of adhesive properties to enamel, the monomer (g-1) having an acidic group is preferably a monomer having a monofunctional acidic group with any one of an acryloyl group, a methacryloyl group, an acrylamide group, and a methacrylamide group. Specific examples are as follows.

Examples of the monomer having a phosphoric acid group include 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxydecyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis[2-(meth)acryloyloxyethyl]hydrogen phosphate, bis[4-(meth)acryloyloxybutyl]hydrogen phosphate, bis[6-(meth)acryloyloxyhexyl]hydrogen phosphate, bis[8-(meth)acryloyloxyoctyl]hydrogen phosphate, bis[9-(meth)acryloyloxynonyl]hydrogen phosphate, bis[10-(meth)acryloyloxydecyl]hydrogen phosphate, 1,3-di(meth)acryloyloxypropyl dihydrogen phosphate, 2-(meth)acryloyloxyethylphenyl hydrogen phosphate, 2-(meth)acryloyloxyethyl-(2-bromoethyl)hydrogen phosphate, 2-(meth)acryloyloxyethyl-(4-methoxyphenyl)hydrogen phosphate, 2-(meth)acryloyloxypropyl-(4-methoxyphenyl)hydrogen phosphate, and amine salts of these.

Examples of the monomer having a pyrophosphoric acid group include bis[2-(meth)acryloyloxyethyl]pyrophosphate, bis[4-(meth)acryloyloxybutyl]pyrophosphate, bis[6-(meth)acryloyloxyhexyl]pyrophosphate, bis[8-(meth)acryloyloxyoctyl]pyrophosphate, bis[10-(meth)acryloyloxydecyl]pyrophosphate, and amine salts of these.

Examples of the monomer having a thiophosphoric acid group include 2-(meth)acryloyloxyethyl dihydrogen thiophosphate, 3-(meth)acryloyloxypropyl dihydrogen thiophosphate, 4-(meth)acryloyloxybutyl dihydrogen thiophosphate, 5-(meth)acryloyloxypentyl dihydrogen thiophosphate, 6-(meth)acryloyloxyhexyl dihydrogen thiophosphate, 7-(meth)acryloyloxyheptyl dihydrogen thiophosphate, 8-(meth)acryloyloxyoctyl dihydrogen thiophosphate, 9-(meth)acryloyloxynonyl dihydrogen thiophosphate, 10-(meth)acryloyloxydecyl dihydrogen thiophosphate, 11-(meth)acryloyloxyundecyl dihydrogen thiophosphate, 12-(meth)acryloyloxydodecyl dihydrogen thiophosphate, 16-(meth)acryloyloxyhexadecyl dihydrogen thiophosphate, 20-(meth)acryloyloxyicosyl dihydrogen thiophosphate, and amine salts of these.

Examples of the monomer having a phosphonic acid group include 2-(meth)acryloyloxyethylphenyl phosphonate, 5-(meth)acryloyloxypentyl-3-phosphonopropionate, 6-(meth)acryloyloxyhexyl-3-phosphonopropionate, 10-(meth)acryloyloxydecyl-3-phosphonopropionate, 6-(meth)acryloyloxyhexyl phosphonoacetate, and 10-(meth)acryloyloxydecyl phosphonoacetate, and amine salts of these.

Examples of the monomer having a carboxylic acid group include (meth)acrylic acid, 4-(meth)acryloyloxyethoxycarbonyl phthalic acid; 4-(meth)acryloyloxyethyl trimellitic acid, 4-(meth)acryloyloxybutyloxycarbonyl phthalic acid, 4-(meth)acryloyloxyhexyloxycarbonyl phthalic acid, 4-(meth)acryloyloxyoctyloxycarbonyl phthalic acid, 4-(meth)acryloyloxydecyloxycarbonyl phthalic acid, and acid anhydrides of these; and 5-(meth)acryloylaminopentyl carboxylic acid, 6-(meth)acryloyloxyhexane-1,1-dicarboxylic acid, 8-(meth)acryloyloxyoctane-1,1-dicarboxylic acid, 10-(meth)acryloyloxydecane-1 , 1 -dicarboxylic acid, 11-(meth)acryloyloxyundecane-1 ,1 - dicarboxylic acid, and amine salts of these.

Examples of the monomer having a sulfonic acid group include 2-(meth)acrylamide-2-methylpropane sulfonic acid, 2-sulfoethyl (meth)acrylate, and amine salts of these.

Preferred among these examples of monomers (g-1) having an acidic group are monomers having a phosphoric acid group, a pyrophosphoric acid group, or a carboxylic acid because such monomers show even superior adhesion to tooth structure. Particularly preferred are monomers having a phosphoric acid group. The monomers having a phosphoric acid group are more preferably monomers having a divalent phosphoric acid group with a C3 to C20 alkyl group or alkylene group constituting the backbone within the molecule, even more preferably monomers having a divalent phosphoric acid group with a C6 to C12 alkylene group constituting the backbone within the molecule, such as 10-methacryloyloxydecyl dihydrogen phosphate.

The monomer (g-1) having an acidic group may be contained alone, or two or more thereof may be contained in combination. The content of monomer (g-1) having an acidic group is not particularly limited, as long as the present invention can exhibit its effects. However, the content of monomer (g-1) having an acidic group ranges preferably from 0.1 to 30 parts by mass, more preferably from 0.5 to 20 parts by mass, even more preferably from 1.0 to 15 parts by mass, particularly preferably from 2.0 to 8.0 parts by mass in total 100 parts by mass of the dental cleaning material (Y). When the content of monomer (g-1) having an acidic group is at or above these lower limits, it is easier to produce the effect of adding monomer (g-1) having an acidic group. When the content of monomer (g-1) having an acidic group is at or below the foregoing upper limits, it is possible to reduce the residual presence of monomer (g-1) having an acidic group on tooth structure, allowing the self-adhesive dental composite resin (X) to retain its curing properties after its application following treatment with the dental cleaning material (Y), thus providing good adhesive properties.

Aside from the monomer (g-1) having an acidic group, the dental cleaning material (Y) may additionally comprise a monomer (b) having no acidic group. The monomer (b) having no acidic group is the same as that of the self-adhesive dental composite resin (X). Examples of the monomer (b) having no acidic group include the hydrophobic monomer (b-2) having no acidic group, and the hydrophilic monomer (b-3) having no acidic group used for the self-adhesive dental composite resin (X). In view of the cleaning performance of dental cleaning material (Y), the dental cleaning material (Y) is preferably one that is essentially free of a monomer (b) having no acidic group. A certain preferred embodiment is, for example, a dental cleaning material (Y) that comprises an organic amine compound (f-1), a monomer (g-1) having an acidic group, and water (h), and that is essentially free of a monomer (b) having no acidic group. Here, "being essentially free of a monomer (b) having no acidic group" means that the content of monomer (b) having no acidic group is less than 5.0 mass%, preferably less than 1.0 mass%, more preferably less than 0.1 mass% in the total amount of monomers contained in the dental cleaning material (Y).

### · Organic Compound (g-2) Having no Polymerizable Group but Having Acidic Group

The organic compound (g-2) having no polymerizable group but having an acidic group may be an organic compound having the same acidic group (e.g., a phosphoric acid group, a phosphonic acid group, a pyrophosphoric acid group, a carboxylic acid group, or a sulfonic acid group) as that of the acidic monomer (g-1), or may be an anionic surfactant. For the same reason described for the monomer (g-1) having an acidic group, the organic compound having the same acidic group (e.g., a phosphoric acid group, a phosphonic acid group, a pyrophosphoric acid group, a carboxylic acid group, or a sulfonic acid group) as that of the acidic monomer (g-1) is more preferably a divalent phosphoric acid group-containing compound having a C3 to C20 alkyl group or alkylene group constituting the backbone within the molecule, even more preferably a divalent phosphoric acid group-containing compound having a C6 to C12 alkylene group constituting the backbone within the molecule. Examples of the divalent phosphoric acid group-containing compound having a C3 to C20 alkyl group or alkylene group constituting the backbone within the molecule include hexylphosphoric acid, heptylphosphoric acid, octylphosphoric acid, nonylphosphoric acid, decylphosphoric acid, undecylphosphoric acid, and dodecylphosphoric acid.

Specific examples of the anionic surfactant include higher fatty acids, alkyl sulfates, alkyl sulfonates, and alkylaryl sulfonates.

Examples of the higher fatty acids include C8 to C20 fatty acids such as caproic acid, caprylic acid, capric acid, lauric acid, myristic acid, palmitic acid, stearic acid, and oleic acid.

Examples of the alkyl sulfates include C6 to C18 lithium alkyl sulfates such as lithium hexyl sulfate, lithium octyl sulfate, lithium decyl sulfate, lithium dodecyl sulfate, lithium tetradecyl sulfate, lithium hexadecyl sulfate, and lithium octadecyl sulfate.

Examples of the alkyl sulfonates include C6 to C18 alkyl sulfonates such as hexyl sulfonate, octyl sulfonate, decyl sulfonate, dodecyl sulfonate, tetradecyl sulfonate, hexadecyl sulfonate, and octadecyl sulfonate.

Examples of the alkylaryl sulfonates include alkylaryl sulfonates having 6 to 18 carbon atoms (excluding those in the aromatic ring), such as hexyl benzenesulfonate, octyl benzenesulfonate, decyl benzenesulfonate, dodecyl benzenesulfonate, tetradecyl benzenesulfonate, hexadecyl benzenesulfonate, and octadecyl benzenesulfonate.

Considering the enhanced effectiveness to reduce precipitation upon exposure to air before use, preferred among these anionic surfactants are alkyl sulfates having 6 to 18 carbon atoms, more preferably alkyl sulfates having 8 to 16 carbon atoms.

The organic compound (g-2) having no polymerizable group but having an acidic group may be contained alone, or two or more thereof may be contained in combination. The content of organic compound (g-2) having no polymerizable group but having an acidic group is not particularly limited, as long as the present invention can exhibit its effects. However, the content of organic compound (g-2) having no polymerizable group but having an acidic group ranges preferably from 0.1 to 30 parts by mass, more preferably from 0.5 to 20 parts by mass, even more preferably from 1.0 to 15 parts by mass, particularly preferably from 2.0 to 8.0 parts by mass in total 100 parts by mass of dental cleaning material (Y). When the content of organic compound (g-2) having no polymerizable group but having an acidic group is at or above these lower limits, it is easier to produce the effect of adding organic compound (g-2) having no polymerizable group but having an acidic group. When the content of organic compound (g-2) having no polymerizable group but having an acidic group is at or below the foregoing upper limits, it is possible to reduce the significant residual presence of organic compound (g-2) having no polymerizable group but having an acidic group in cavities, allowing the self-adhesive dental composite resin (X) to retain its curing properties after its application following treatment with the dental cleaning material (Y), thus providing good cavity sealing properties.

The anionic component (g) may be contained alone, or two or more thereof may be contained in combination.

### <Water (h)>

Water (h) is a solvent for dissolving the cationic component (f) and anionic component (g) in dental cleaning material (Y). Water (h) is also necessary for dental cleaning material (Y) to exhibit its ability to remove the smear layer and contamination due to protein-containing bodily fluids. It is required that water (h) be essentially free of impurities that have a negative impact on the adhesive properties. Preferably, water (h) is distilled water or ion-exchange water. The content of water (h) ranges preferably from 40 to 99.8 parts by mass, more preferably from 50 to 99 parts by mass, even more preferably from 60 to 98 parts by mass in total 100 parts by mass of the dental cleaning material (Y).

A dental cleaning material (Y) according to the present invention may additionally comprise a solvent other than water. By containing a solvent other than water, it may be possible to achieve a more efficient cleaning effect through viscosity adjustment or concentration of the active ingredients in the dental cleaning material (Y). Examples of solvents other than water include organic solvents such as acetone and ethyl methyl ketone; and alcohols such as ethanol, 1-propanol, 2-propanol, 2-methyl-2-propanol, glycerin, diglycerin, polyglycerin, propylene glycol, dipropylene glycol, polypropylene glycol, ethylene glycol, diethylene glycol, polyethylene glycol, polyethylene glycol monomethyl ether, 1,2-pentadiol, 1,2-hexanediol, and 1,2-octanediol. The content of solvents other than water (h) is not particularly limited, and is preferably 50 parts or less by mass, more preferably 40 parts or less by mass, even more preferably 20 parts or less by mass in total 100 parts by mass of the dental cleaning material (Y). The content of solvents other than water (h) is preferably 100 parts or less by mass, more preferably 85 parts or less by mass, or may be 60 parts or less by mass, relative to 100 parts by mass of water (h).

A dental cleaning material (Y) of the present invention must have a pH of 2.0 or more and less than 9.0, as noted above. Preferably, the upper limit of pH is less than 8.0. With an upper limit pH of less than 8.0, the dental cleaning material (Y) causes even less demineralization of the tooth structure itself, achieving a further reduction of a decrease of adhesive properties, and providing an even safer dental cleaning material (Y) having excellent bond strength and cavity sealing properties on tooth structure. In this respect, the pH of dental cleaning material (Y) is preferably 2.0 or more and less than 8.0, more preferably 2.5 or more and 7.5 or less, even more preferably 3.0 or more and 6.5 or less, particularly preferably 3.5 or more and 5.5 or less. Provided that the pH is confined within these preferred pH ranges, a certain preferred embodiment is, for example, a dental cleaning material (Y) having a pH of 3.6 or more and 6.8 or less. The pH can be measured with a known measurement device, for example, such as LAQUAtwin manufactured by Horiba Ltd.

### . Gelatinizer

A dental cleaning material (Y) of the present invention preferably comprises a gelatinizer. The gelatinizer may be a hydrophilic filler, a hydrophobic filler, or a polymeric thickener. The gelatinizer may be used alone, or two or more thereof may be used in combination. In a certain preferred embodiment of the present invention, the dental cleaning material (Y) comprises a hydrophilic filler and a hydrophobic filler as gelatinizers. In another preferred embodiment, the dental cleaning material (Y) solely comprises a polymeric thickener as a gelatinizer. In yet another preferred embodiment, the dental cleaning material (Y) comprises a hydrophilic filler, a hydrophobic filler, and a polymeric thickener. Preferably, the dental cleaning material (Y) comprises a gelatinizer combining all of a hydrophilic filler, a hydrophobic filler, and a polymeric thickener. By using a hydrophilic filler, a hydrophobic filler, and a polymeric thickener in combination, the dental cleaning material (Y) can have improved spreadability on tooth structure, and becomes less runny by undergoing gelation, producing the effect to remove only the smear layer without inhibiting the surfactant action. When serving as gelatinizers, the hydrophilic filler and hydrophobic filler used as gelatinizers may be any of the fillers exemplified as filler (d).

The hydrophilic filler means a filler with unhydrophobized particle surfaces. For example, when the hydrophilic filler is a particle having surface silanol groups (such as silica), the hydrophilic filler means a particle with untreated surface silanol groups. The hydrophobic filler means a filler with hydrophobized particle surfaces. For example, when the hydrophobic filler is a particle with surface silanol groups (such as silica), the hydrophobic filler means a particle with the surface silanol groups hydrophobized with a substituent such as dimethylsilyl, trimethylsilyl, dimethylpolysiloxane, dimethylsiloxane, aminoalkylsilyl, methacrylsilyl, or alkylsilyl. The hydrophilic filler is preferably a hydrophilic inorganic filler, more preferably hydrophilic fumed silica. The hydrophobic filler is preferably a hydrophobic inorganic filler, more preferably hydrophobic fumed silica. The average particle diameters of hydrophilic filler and hydrophobic filler are not particularly limited, and are preferably 0.0001 to 50 µm, more preferably 0.001 to 10 µm. In view of providing even superior spreadability to dental cleaning material (Y), the average particle diameters of hydrophilic filler and hydrophobic filler are even more preferably 0.001 to 1.0 µm.

The average particle diameters of hydrophilic filler and hydrophobic filler are average primary particle diameters, and can be determined by a dynamic light scattering method, a laser diffraction scattering method, or electron microscopy of particles. Specifically, a laser diffraction scattering method is more convenient for the measurement of particle diameters of 0.1 µm or more, whereas a dynamic light scattering method and electron microscopy are more convenient for the measurement of ultrafine particles of less than 0.1 µm. Here, 0.1 µm is a measured value by a laser diffraction scattering method. As a specific example of a laser diffraction scattering method, the average particle diameter can be measured by volume using a laser diffraction particle size distribution analyzer (SALD-2300 manufactured by Shimadzu Corporation), using a 0.2% sodium hexametaphosphate aqueous solution as dispersion medium.

A scanning electron microscope (e.g., SU3800, S-4000, manufactured by Hitachi High-Technologies Corporation) can be used for electron microscopy. In electron microscopy, for example, particles may be photographed with an electron microscope, and the size of particles (at least 200 particles) observed in a unit field of the micrograph may be measured using image-analyzing particle-size-distribution measurement software (Mac-View manufactured by Mountech Co., Ltd.). Here, the particle diameter is determined as an arithmetic mean value of the maximum and minimum lengths of particles, and the average primary particle diameter is calculated from the number of particles and the particle diameter. In the measurement of average particle diameter based on a dynamic light scattering method, for example, an average of particle sizes measured with a concentrated particle size analyzer (FPAR 1000 manufactured by Otsuka Electronics Co., Ltd.; laser beam wavelength: 650 nm) is represented as the DLS particle size. The measurement can be conducted by volume with a 0.2% sodium hexametaphosphate aqueous solution used as dispersion medium.

Examples of the hydrophilic filler include hydrophilic fumed silica (particulate silica manufactured by Nippon Aerosil Co., Ltd., including AEROSIL^{®} 90, AEROSIL^{®} 130, AEROSIL^{®} 150, AEROSIL^{®} 200, AEROSIL^{®} 255, AEROSIL^{®} 300, AEROSIL^{®} 380, AEROSIL^{®} OX50, AEROSIL^{®} TT600, AEROSIL^{®} 200 F, AEROSIL^{®} 380 F, AEROSIL^{®} 200 Pharma, and AEROSIL^{®} 300 Pharma. Preferred is AEROSIL^{®} 130.

Examples of the hydrophobic filler include hydrophobic fumed silica (particulate silica manufactured by Nippon Aerosil Co., Ltd.), including AEROSIL^{®} R972, AEROSIL^{®} R974, AEROSIL^{®} R104, AEROSIL^{®} R106, AEROSIL^{®} R202, AEROSIL^{®} R208, AEROSIL^{®} R805, AEROSIL^{®} R812, AEROSIL^{®} R812 S, AEROSIL^{®} R816, AEROSIL^{®} R7200, AEROSIL^{®} R8200, AEROSIL^{®} R9200, AEROSIL^{®} R711, AEROSIL^{®} R50, AEROSIL^{®} NY50, AEROSIL^{®} NY50 L, AEROSIL^{®} NY200, AEROSIL^{®} RY200 S, AEROSIL^{®} RX50, AEROSIL^{®} RX, AEROSIL^{®} RX00, AEROSIL^{®} R504, AEROSIL^{®} RNX90 S, AEROSIL^{®} NX90 G, AEROSIL^{®} RY300, AEROSIL^{®} REA90, AEROSIL^{®} REA200, AEROSIL^{®} RY51, AEROSIL^{®} NA50 Y, AEROSIL^{®} RA200 HS, AEROSIL^{®} NA50 H, AEROSIL^{®} NA130 K, AEROSIL^{®} NA200 Y, AEROSIL^{®} NX130, AEROSIL^{®} RY200 L, AEROSIL^{®} R709, and AEROSIL^{®} R976 S. Preferred is AEROSIL^{®} R972.

The polymeric thickener refers to a polymer added to materials to adjust viscosity, in order to enhance dispersibility and viscosity. The polymeric thickener may be a natural substance or a synthetic substance. Examples of the natural substance include casein, methyl cellulose, carboxymethyl cellulose, and hydroxyethyl cellulose. Examples of the synthetic substance include polyvinyl alcohol, sodium polyacrylate, polyethylene oxide, polyethylene glycol, and polyvinylpyrrolidone. The polymeric thickener may be a commercially available product, for example, such as Macrogol 4000 (a polyethylene glycol-based thickener manufactured by Maruishi Pharmaceutical. Co., Ltd.), and Macrogol 400 (a polyethylene glycol-based thickener manufactured by Maruishi Pharmaceutical. Co., Ltd.), or polyvinylpyrrolidone (manufactured by IPS Corporation). Preferred is Macrogol 4000 (manufactured by Maruishi Pharmaceutical. Co., Ltd.).

The gelatinizer content is not particularly limited, as long as the present invention can exhibit its effects. However, the gelatinizer content ranges preferably from 0.50 to 40 parts by mass, more preferably from 0.80 to 35 parts by mass, even more preferably from 1 to 30 parts by mass, particularly preferably from 4 to 25 parts by mass in total 100 parts by mass of the dental cleaning material (Y). Preferably, the hydrophilic filler is hydrophilic fumed silica, and the hydrophobic filler is hydrophobic fumed silica. In view of inhibiting the separation of the gel component and liquid component contained in the dental cleaning material (Y), the mass ratio of the hydrophilic filler and the hydrophobic filler is preferably 4:6 to 2:8, more preferably 4.3:5.7 to 2.2:7.8. In view of inhibiting the separation of the gel component and liquid component contained in the dental cleaning material (Y), the content of the polymeric thickener ranges preferably from 1 to 25 parts by mass, more preferably from 4 to 20 parts by mass, even more preferably from 8 to 17 parts by mass in total 100 parts by mass of the dental cleaning material (Y).

The dental cleaning material (Y) may additionally comprise known components, such as a polymerization initiator, a polymerization accelerator, a fluorine-ion releasing substance, and additives (a pH adjuster, a chelating agent, a polymerization inhibitor (for example, dibutylhydroxytoluene (BHT), hydroquinonemonomethyl ether (MEHQ)), a colorant, a fluorescent agent, a fragrance) to such an extent that it does not hinder the effectiveness of the present invention. It is also possible to add antimicrobial substances such as cetylpyridinium chloride, benzalkonium chloride, (meth)acryloyloxydodecylpyridinium bromide, (meth)acryloyloxyhexadecylpyridinium chloride, (meth)acryloyloxydecylammonium chloride, and triclosan. In view of the cleaning performance of dental cleaning material (Y), it is preferable in certain embodiments that the dental cleaning material (Y) be essentially free of a polymerization initiator. Here, "being essentially free of a polymerization initiator" means that the content of polymerization initiator is less than 0.1 parts by mass, preferably less than 0.05 parts by mass, more preferably less than 0.01 parts by mass in the total amount of monomers contained in the dental cleaning material (Y).

A certain preferred embodiment (W-1) is, for example, a dental cleaning material (Y) that comprises an organic amine compound (f-1), a monomer (g-1) having an acidic group, and water (h), in which the monomer (g-1) having an acidic group comprises at least one monomer selected from the group consisting of a monomer having a phosphoric acid group, a monomer having a pyrophosphoric acid group, and a monomer having a carboxylic acid group, with the content of the organic amine compound (f-1) ranging from 0.1 to 20 parts by mass, the content of the monomer (g-1) having an acidic group ranging from 0.1 to 30 parts by mass, and the content of water (h) ranging from 40 to 99.8 parts by mass in total 100 parts by mass of the dental cleaning material (Y).

Another preferred embodiment (W-2) is, for example, a dental cleaning material (Y) that comprises an organic amine compound (f-1), a monomer (g-1) having an acidic group, and water (h), in which the organic amine compound (f-1) comprises a tertiary organic amine compound, and the monomer (g-1) having an acidic group comprises at least one monomer selected from the group consisting of a monomer having a phosphoric acid group, a monomer having a pyrophosphoric acid group, and a monomer having a carboxylic acid group, with the content of the organic amine compound (f-1) ranging from 0.1 to 20 parts by mass, the content of the monomer (g-1) having an acidic group ranging from 0.1 to 30 parts by mass, and the content of water (h) ranging from 40 to 99.8 parts by mass in total 100 parts by mass of the dental cleaning material (Y).

Yet another preferred embodiment (W-3) is, for example, a dental cleaning material (Y) that comprises an organic amine compound (f-1), a monomer (g-1) having an acidic group, and water (h), in which the organic amine compound (f-1) comprises a tertiary organic amine compound with an aliphatic compound contained in the tertiary organic amine compound, and the monomer (g-1) having an acidic group comprises at least one monomer selected from the group consisting of a monomer having a phosphoric acid group, a monomer having a pyrophosphoric acid group, and a monomer having a carboxylic acid group, with the content of the organic amine compound (f-1) ranging from 0.1 to 20 parts by mass, the content of the monomer (g-1) having an acidic group ranging from 0.1 to 30 parts by mass, and the content of water (h) ranging from 40 to 99.8 parts by mass in total 100 parts by mass of the dental cleaning material (Y).

Still another preferred embodiment (W-4) is, for example, a dental cleaning material (Y) that comprises an organic amine compound (f-1), a monomer (g-1) having an acidic group, and water (h), in which the organic amine compound (f-1) comprises a tertiary organic amine compound with an aliphatic compound and an aromatic compound contained in the tertiary organic amine compound, and the monomer (g-1) having an acidic group comprises at least one monomer selected from the group consisting of a monomer having a phosphoric acid group, a monomer having a pyrophosphoric acid group, and a monomer having a carboxylic acid group, with the content of the organic amine compound (f-1) ranging from 0.1 to 20 parts by mass, the content of the monomer (g-1) having an acidic group ranging from 0.1 to 30 parts by mass, and the content of water (h) ranging from 40 to 99.8 parts by mass in total 100 parts by mass of the dental cleaning material (Y).

Yet another preferred embodiment (W-5) is, for example, a dental cleaning material (Y) that comprises an organic amine compound (f-1), a monomer (g-1) having an acidic group, and water (h), in which the organic amine compound (f-1) comprises a tertiary organic amine compound with an aliphatic compound having two or more hydroxyl groups contained in the tertiary organic amine compound, and the monomer (g-1) having an acidic group comprises a monomer having a phosphoric acid group, with the content of the organic amine compound (f-1) ranging from 0.1 to 20 parts by mass, the content of the monomer (g-1) having an acidic group ranging from 0.1 to 30 parts by mass, and the content of water (h) ranging from 40 to 99.8 parts by mass in total 100 parts by mass of the dental cleaning material (Y).

Still another preferred embodiment (W-6) is, for example, a dental cleaning material (Y) that comprises an organic amine compound (f-1), a monomer (g-1) having an acidic group, and water (h), in which the organic amine compound (f-1) comprises a tertiary organic amine compound with an aliphatic compound and an aromatic compound contained in the tertiary organic amine compound, where the aliphatic compound is an aliphatic compound having two or more hydroxyl groups, and the aromatic compound is an aromatic compound having two or more hydroxyl groups, and in which the monomer (g-1) having an acidic group is at least one selected from the group consisting of a monomer having a phosphoric acid group, a monomer having a pyrophosphoric acid group, and a monomer having a carboxylic acid group, with the content of the organic amine compound (f-1) ranging from 0.1 to 20 parts by mass, the content of the monomer (g-1) having an acidic group ranging from 0.1 to 30 parts by mass, and the content of water (h) ranging from 40 to 99.8 parts by mass in total 100 parts by mass of the dental cleaning material (Y).

Yet another preferred embodiment (W-7) is, for example, a dental cleaning material (Y) of any one of the foregoing embodiments (W-1) to (W-6) that additionally comprises a basic inorganic compound (f-2), with the total content of the organic amine compound (f-1) and basic inorganic compound (f-2) ranging from 0.1 to 20 parts by mass, the content of the monomer (g-1) having an acidic group ranging from 0.1 to 30 parts by mass, and the content of water (h) ranging from 40 to 99.8 parts by mass in total 100 parts by mass of the dental cleaning material (Y).

Still another preferred embodiment (W-8) is, for example, a dental cleaning material (Y) of any one of the foregoing embodiments (W-1) to (W-7) that comprises a gelatinizer, with the content of the organic amine compound (f-1) ranging from 0.1 to 20 parts by mass, the content of the monomer (g-1) having an acidic group ranging from 0.1 to 30 parts by mass, the content of water (h) ranging from 45 to 99.3 parts by mass, and the content of the gelatinizer ranging from 0.50 to 40 parts by mass.

In all of the preferred embodiments (W-1) to (W-8) above, the type and content of each component may be appropriately varied, and changes such as addition and deletion of any component may be made following the descriptions of the present specification.

A feature of a method of production of a dental cleaning material (Y) of the present invention is that the method comprises the step of mixing a monomer (g-1) having an acidic group, water (h), and an organic amine compound (f-1). The methods and devices used for mixing are not particularly limited, as long as the present invention can exhibit its effect. When the monomer (g-1) having an acidic group is poorly soluble in water (h), the method preferably comprises mixing the monomer (g-1) having an acidic group after mixing the organic amine compound (f-1) with water (h), or mixing water (h) after mixing the organic amine compound (f-1) with the monomer (g-1) having an acidic group. When the dental cleaning material (Y) comprises additional components (for example, a gelatinizer) other than the components (f) to (h), such additional components may be mixed simultaneously with the components (f) to (h), or an additional step of mixing the additional components may be incorporated separately from the step of mixing the components (f) to (h).

### <Configuration of Dental Filling Kit of Present Invention>

A dental filling kit of the present invention comprises a self-adhesive dental composite resin (X) and a dental cleaning material (Y) as its components. Normally, these are separately filled in independent containers.

A dental filling kit of the present invention may additionally incorporate components such as an etchant (C), a dental primer (D), and a dental bonding material (E). In view of cavity sealing properties, it is preferable that the etchant (C) be applied to only the enamel. In view of the bond strength to tooth structure and cavity sealing properties, it is preferable to incorporate a dental primer (D) and a dental bonding material (E), more preferably a dental bonding material (E). The dental primer (D) and dental bonding material (E) may or may not be polymerized alone. However, in view of cavity sealing properties, it is preferable to polymerize these components by photopolymerization or chemical polymerization. The etchant (C), dental primer (D), and dental bonding material (E) may be used alone, or may be used in combination. In view of cavity sealing properties, it is preferable to employ a procedure that applies the dental primer (D) after using the etchant (C), before applying the dental bonding material (E).

### <Specific Method and Procedure of Application>

### · Treatment with Dental Cleaning Material (Y)

The dental cleaning material (Y) is used to treat the surface of tooth structure where the self-adhesive dental composite resin (X) will be applied. The surface of tooth structure may be treated using various methods, including, for example, a method that rinses off the dental cleaning material (Y) with water after it is applied and left to stand for a predetermined treatment time, a method that rinses off the dental cleaning material (Y) with water after it is applied and rubbed for a predetermined treatment time with an instrument such as a microbrush, and a method that wipes off the dental cleaning material (Y) with a material such as a dry or wet swab after the dental cleaning material (Y) is applied and rubbed for a predetermined treatment time with an instrument such as a microbrush. However, in view of cavity cleanness, it is preferable to use the method that rinses off the dental cleaning material (Y) with water after it is applied and rubbed for a predetermined treatment time with an instrument such as a microbrush. After the dental cleaning material (Y) is rinsed off with water, the applied areas are dried by air or with a swab, for example.

### · Application of Self-Adhesive Dental Composite Resin (X)

The self-adhesive dental composite resin (X) is applied after the tooth surface (cavities) is treated with the dental cleaning material (Y). After placing the self-adhesive dental composite resin (X) and shaping the surface, the self-adhesive dental composite resin (X) is cured by photoirradiation with a dental photoirradiator when the self-adhesive dental composite resin (X) is one containing the photopolymerization initiator (c-1). In the case of a self-adhesive dental composite resin (X) containing a chemical polymerization initiator, the self-adhesive dental composite resin (X) is left to stand until curing proceeds to completion. Depending on circumstances, this is followed by modifying the surface shape, and polishing the surface.

When components such as the etchant (C), dental primer (D), and dental bonding material (E) are incorporated, the self-adhesive dental composite resin (X) is used before or after the application of dental cleaning material (Y) when the etchant (C) is used, whereas the self-adhesive dental composite resin (X) is used after the application of dental cleaning material (Y) when the dental primer (D) or dental bonding material (E) is used.

### EXAMPLES

The following describes the present invention in greater detail using Examples and Comparative Examples. However, the present invention is not limited to the following descriptions. In the following, "part(s)" means "part(s) by mass", unless otherwise specifically stated.

The components of the dental filling kits of Examples and Comparative Examples are presented below, along with the abbreviations.

### · Self-Adhesive Dental Composite Resin (X)

### [Monomer (a) Having Acidic Group]

MDP: 10-methacryloyloxydecyl dihydrogen phosphate

### [Monomer (b) Having no Acidic Group]

Bis-GMA: 2,2-bis[4-(3-methacryloyloxy-2-hydroxypropoxy)phenyl]propane
D-2.6E: 2,2-bis(4-methacryloyloxypolyethoxyphenyl)propane (the average number of moles of ethoxy group added: 2.6)
3G: triethylene glycol dimethacrylate
DD: 1,10-decanediol dimethacrylate
MAEA: N-methacryloyloxyethylacrylamide
DEAA: N,N-diethylacrylamide
HEMA: 2-hydroxyethyl methacrylate

### [Photopolymerization Initiator (c-1)]

- Water-soluble photopolymerization initiator (c-1a)
   Li-TPO: lithium phenyl(2,4,6-trimethylbenzoyl)phosphinate
- Water-insoluble photopolymerization initiator (c-1b)
   CQ: camphorquinone
   BAPO: bis(2,4,6-trimethylbenzoyl)phenylphosphine oxide

### [Filler (d)]

### Filler 1: Ultrafine particulate silica Aerosil^{®} R 972, manufactured by Nippon Aerosil Co., Ltd.; average particle diameter: 16 nm

### Filler 2: Silane-treated silica

A three-neck flask was charged with 100 g of OX-50 (ultrafine particulate silica Aerosil^{®} OX-50, manufactured by Nippon Aerosil Co., Ltd.; average particle diameter: 0.04 µm), 7 g of γ-methacryloyloxypropyltrimethoxysilane, and 200 mL of a 0.3 mass% aqueous solution of acetic acid, and the mixture was stirred at room temperature for 2 hours. After removing water by freeze drying, the mixture was subjected to a heat treatment at 80°C for 5 hours to obtain a filler 2.

### Filler 3: Silane-treated silica stone powder

A silica stone powder (Hi-Silica, manufactured by Nitchitsu Co., Ltd. under this trade name) was pulverized with a ball mill to obtain a pulverized silica stone powder. The pulverized silica stone powder had an average particle diameter of 2.2 µm as measured by volume with a laser diffraction particle size distribution analyzer (Model SALD-2300, manufactured by Shimadzu Corporation). One-hundred parts by mass of the pulverized silica stone powder was surface treated with four parts by mass of γ-methacryloyloxypropyltrimethoxysilane by an ordinary method to obtain a silane-treated silica stone powder.

### Filler 4: Silane-treated barium glass powder

A barium glass (Product Code E-3000, manufactured by Esstech) was pulverized with a ball mill to obtain a barium glass powder. The barium glass powder had an average particle diameter of 2.4 µm as measured by volume with a laser diffraction particle size distribution analyzer (Model SALD-2300, manufactured by Shimadzu Corporation). One-hundred parts by mass of the barium glass powder was surface treated with three parts by mass of γ-methacryloyloxypropyltrimethoxysilane by an ordinary method to obtain a silane-treated barium glass powder.

### Filler 5: Silane-treated barium glass powder

A three-neck flask was charged with 100 g of GM27884 NF180 Grade (a barium glass manufactured by SCHOTT; average particle diameter: 0.18 µm), 13 g of γ-methacryloyloxypropyltrimethoxysilane, and 200 mL of a 0.3 mass% aqueous solution of acetic acid, and the mixture was stirred at room temperature for 2 hours. After removing water by freeze drying, the mixture was subjected to a heat treatment at 80°C for 5 hours to obtain a filler 5.

### Filler 6: Silane-treated barium glass powder

A three-neck flask was charged with 100 g of 8235 UF0.7 Grade (a barium glass manufactured by SCHOTT; average particle diameter: 0.7 µm), 6 g of γ-methacryloyloxypropyltrimethoxysilane, and 200 mL of a 0.3 mass% aqueous solution of acetic acid, and the mixture was stirred at room temperature for 2 hours. After removing water by freeze drying, the mixture was subjected to a heat treatment at 80°C for 5 hours to obtain a filler 6.

### Filler 7: Silane-treated spherical silica-titania composite oxide powder

A three-neck flask was charged with 100 g of spherical silica-titania composite oxide (average particle diameter: 0.3 µm), 10 g of γ-methacryloyloxypropyltrimethoxysilane, and 200 mL of a 0.3 mass% aqueous solution of acetic acid, and the mixture was stirred at room temperature for 2 hours. After removing water by freeze drying, the mixture was subjected to a heat treatment at 80°C for 5 hours to obtain a filler 7.

### [Polymerization Accelerator (e)]

DABE: ethyl 4-(N,N-dimethylamino)benzoate

### [Polymerization Inhibitor]

B HT: 3,5-di-t-butyl-4-hydroxytoluene

### · Dental Cleaning Material (Y)

### [Cationic Component (f)]

TTA: triethanolamine
DEPT: N,N-bis(2-hydroxyethyl)-p-toluidine
DMAEMA: 2-(dimethylamino)ethyl methacrylate
Na₂HPO₄: disodium hydrogen phosphate

### [Anionic Component (g)]

MDP: 10-methacryloyloxydecyl dihydrogen phosphate
DDP: dodecylphosphoric acid

### [Gelatinizer]

AEROSIL 130: particulate silica AEROSIL^{®} 130 manufactured by Nippon Aerosil Co., Ltd. (hydrophilic fumed silica, average particle diameter: 16 nm)
AEROSIL R972: particulate silica AEROSIL^{®} R972 manufactured by Nippon Aerosil Co., Ltd. (hydrophobic fumed silica, average particle diameter: 16 nm)
Polyvinylpyrrolidone (manufactured by IPS Corporation)
Macrogol 4000 (polyethylene glycol 4000 manufactured by Maruishi Pharmaceutical. Co., Ltd.)

### [Examples 1 to 29 and Comparative Examples 1 to 3]

### [Preparation of Self-Adhesive Dental Composite Resin (X)]

The raw materials shown in Tables 2 to 4 were mixed and kneaded at ordinary temperature (23°C) in the dark to prepare paste-like self-adhesive dental composite resins, and the resin properties were examined following the methods of Test Examples 1 and 2 below. The results are presented in Tables 2 to 4.

### [Preparation of Dental Cleaning Material (Y)]

The components shown in Table 1 were mixed at ordinary temperature (23°C) to prepare dental cleaning materials (Y). The dental cleaning materials (Y) were used after being filled into the containers of Teethmate^{®} Desensitizer, liquid formulation (manufactured by Kuraray Noritake Dental Inc.).

### Test Example 1: Bond Strength to Dentin

### [Shear Adhesion Test on Dentin]

The labial surface of a bovine mandibular incisor was ground with #80 silicon carbide paper (manufactured by Nihon Kenshi Co., Ltd.) under running water to obtain a sample with an exposed flat dentin surface. The sample tooth was secured to a tape attached to the bottom of a mold having 15 holes (a 15-hole mold, manufactured by Ultradent Products Inc.; 35 mm in diameter × 25 mm in height). After filling a plaster into the mold, the plaster was left to stand for about 30 minutes to harden. The sample was taken out of the mold, and polished with #600 silicon carbide paper (manufactured by Nihon Kenshi Co., Ltd.) under running water until the polished surface was large enough to provide a bonding surface (Ø = 2.38 mm or more). The bonding surface was then ultrasonically washed with water for 5 minutes.

The dental cleaning material was placed on a dappen dish, applied to the bonding surface with a microbrush, and rubbed for 10 seconds. After application, the dental cleaning material was rinsed off with water. Cleaned samples were prepared in this fashion, whereas uncleaned samples were prepared by not applying the dental cleaning material to the bonding surface.

Subsequently, a separately prepared CR filling mold (Bonding Mold Insert, manufactured by Ultradent Products Inc.; Ø = 2.38 mm) was installed on a dedicated instrument (Bonding Clamp, manufactured by Ultradent Products Inc.). The sample was secured by lowering the CR filling mold, allowing the CR filling mold installed on the dedicated instrument to contact the bonding surface of the sample.

Thereafter, the dental composite resin prepared in each Example and Comparative Example was filled into the hole of the CR filling mold to form a thin layer of no greater than 1 mm.

After filling another portion into the mold (to about 2/3 of the mold, or about 2 mm thick), the self-adhesive dental composite resin was left to stand for 10 seconds, and irradiated with light for 10 seconds using a dental LED photoirradiator (manufactured by Ultradent Products Inc. under the trade name VALO) to cure. The sample was removed from the mold, and used as an adhesion test sample.

The adhesion test sample was immersed in distilled water inside the sample container, and was left to stand for 24 hours in this state in a thermostatic chamber with the chamber temperature set to 37°C. The sample was measured for its bond strength after being taken out of the chamber.

For the measurement of bond strength (shear bond strength), the adhesion test sample was installed on a dedicated holder (Test Base Clamp, manufactured by Ultradent Products Inc.), and the bond strength was measured using a dedicated jig (Crosshead Assembly, manufactured by Ultradent Products Inc.) and a universal testing machine (manufactured by Shimadzu Corporation) with the crosshead speed set at 1 mm/min. The mean values are presented in the tables (n = 10).

### Test Example 2: Cavity Sealing Properties

The surface of a bovine tooth was ground with #80 silicon carbide paper (manufactured by Nihon Kenshi Co., Ltd.) under running water to obtain a sample with an exposed flat enamel surface. A cylindrical cavity, measuring 4 mm in diameter and 2 mm in depth, was then formed with a dental diamond bur (manufactured by Mani Inc. under the trade name Mani Dia-Bur SF-21) by using an air turbine while applying water. Once formed, the cavity interior was rinsed with a stream of water, and was dried by blowing air. Thereafter, the dental cleaning material was applied to the interior of the cavity with a microbrush, and rubbed inside the cavity for 10 seconds to prepare a cleaned sample. Uncleaned samples were also prepared by not applying the dental cleaning material to the bonding surface. After rinsing with water and subsequent air blowing, the self-adhesive dental composite resin was filled into the cavity, and was left to stand for 10 seconds, before irradiating it with a dental LED photoirradiator (manufactured by Ultradent Products Inc. under the trade name VALO) for 10 seconds.

After irradiation, the cleaned samples and uncleaned samples were observed with an optical coherence tomography (OCT) device (IVS-2000 manufactured by Santec Corporation) to evaluate the adequacy of the cavities (n = 3). The cavity sealing properties were rated by giving a score of 0 when no detachment was observed in the cavities, a score of 1 when detachment was observed on the side walls of cavities in any of the samples, a score of 2 when detachment was observed on the cavity floor in any of the samples, and a score of 3 when detachment was observed on the side walls and the floor of cavities in any of the samples.

**[Table 1]**

| | | Cleaning material 1 | Cleaning material 2 | Cleanin 9 material 3 | Cleanin 9 material 4 | Cleaning material 5 | Cleaning material 6 | Cleaning material 7 | Cleaning material 8 | Cleaning material 9 | Cleanin 9 material 10 | Cleaning material 11 | Cleanin 9 material 12 | Cleanin 9 material 13 |
|---|---|---|---|---|---|---|---|---|---|---|---|---|---|---|
| Anionic component (g) | MDP | 5.0 | 5.0 | 4.0 | 4.0 | 4.5 | 3.0 | 5.0 | 4.0 | 4.0 | - | - | 5.0 | - |
| | DDP | - | - | - | - | - | - | - | - | - | 5.0 | 3.0 | - | - |
| Water (h) | Water | 92.0 | 84.0 | 70.0 | 70.0 | 76.0 | 60.0 | 93.0 | 78.0 | 78.0 | 92.0 | 60.0 | 45.0 | 98.0 |
| Cationic component (f) | TTA | - | - | 1.5 | 1.0 | 1.5 | 1.0 | 2.0 | 1.0 | 1.5 | - | 1.0 | - | - |
| | DEPT | - | - | 0.5 | 0.5 | 0.5 | - | - | 0.5 | 0.5 | - | - | - | - |
| | DMAEMA | 2.0 | 2.0 | - | - | - | - | - | - | - | 2.0 | - | - | 2.0 |
| | Na₂HPO₄ | 1.0 | 1.0 | - | 0.5 | 0.5 | - | - | 0.5 | - | 1.0 | - | - | - |
| Organic solvent | Ethanol | - | - | - | - | - | - | - | - | - | - | - | - | - |
| | Acetone | - | - | - | - | - | - | - | - | - | - | - | 50.0 | - |
| Gelatinizer | AEROSIL 130 | - | 3.0 | 3.0 | 3.0 | - | 6.0 | - | - | - | - | 6.0 | - | - |
| | AEROSIL R972 | - | 5.0 | 6.0 | 6.0 | - | 10.0 | - | - | - | - | 10.0 | - | - |
| | Polyvinylpyrrolidone | - | - | - | - | - | 16.0 | - | - | - | - | 16.0 | - | - |
| | Macrogol 4000 | - | - | 15.0 | 15.0 | 17.0 | 4.0 | - | 16.0 | 16.0 | - | 4.0 | - | - |
| pH | | 6.4 | 6.5 | 4.5 | 4.7 | 4.6 | 4.2 | 4.0 | 4.7 | 4.5 | 6.4 | 4.2 | 1.6 | 9.4 |

**[Table 2]**

| Components (parts by mass) | | Ex. 1 | Ex. 2 | Ex. 3 | Ex. 4 | Ex. 5 | Ex. 6 | Ex. 7 | Ex. 8 | Ex. 9 | Ex. 10 | Ex. 11 | Ex. 12 | Ex. 13 | Ex. 14 | Ex. 15 | Ex. 16 |
|---|---|---|---|---|---|---|---|---|---|---|---|---|---|---|---|---|---|
| Cleaning material | | Cleaning material 3 | Cleaning material 3 | Cleaning material 3 | Cleaning material 3 | Cleaning material 3 | Cleaning material 3 | Cleaning material 3 | Cleaning material 3 | Cleaning material 3 | Cleaning material 3 | Cleaning material 3 | Cleaning material 3 | Cleaning material 3 | Cleaning material 3 | Cleaning material 3 | Cleaning material 3 |
| Monomer (a) having acidic group | MDP | 10 | 10 | 10 | 10 | 10 | 10 | 10 | 10 | 10 | 10 | 10 | 10 | 30 | 10 | 10 | 10 |
| Monomer (b) having no acidic group | Bis-GMA | 30 | 30 | 30 | 30 | 30 | 30 | 30 | 30 | 30 | - | 30 | 50 | 30 | - | - | - |
| | D-2.6E | - | - | - | - | - | - | - | - | - | 30 | - | - | - | 30 | 30 | 30 |
| | 3G | 50 | 50 | 50 | 50 | 50 | 50 | 50 | 50 | 50 | 50 | - | 30 | 30 | 40 | 40 | 15 |
| | MAEA | 10 | 10 | 10 | 10 | 10 | 10 | 10 | 10 | 10 | 10 | 10 | 10 | 10 | 10 | 10 | 10 |
| | DD | - | - | - | - | - | - | - | - | - | - | 50 | - | - | - | - | - |
| | HEMA | - | - | - | - | - | - | - | - | - | - | - | - | - | 10 | - | 25 |
| | DEAA | - | - | - | - | - | - | - | - | - | - | - | - | - | - | 10 | - |
| Photopolymerization initiator (c-1) | Li-TPO | - | - | - | - | - | - | - | - | - | - | - | - | - | - | - | |
| | CQ | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 |
| | BAPO | - | - | - | - | - | - | - | - | - | - | - | - | - | - | - | - |
| Filler (d) | Filler 1 | 10 | 10 | 10 | 10 | 10 | - | - | 25 | 40 | 10 | 10 | 10 | 10 | 10 | 10 | 10 |
| | Filler 2 | - | - | - | - | - | - | 10 | - | - | - | - | - | - | - | - | - |
| | Filler 3 | 250 | - | - | - | - | - | 250 | 250 | 250 | 250 | 250 | 250 | 250 | 250 | 250 | 250 |
| | Filler 4 | - | 250 | - | - | - | - | - | - | - | - | - | - | - | - | - | - |
| | Filler 5 | - | - | 180 | - | - | 90 | - | - | - | - | - | - | - | - | - | - |
| | Filler 6 | - | - | - | 200 | - | 100 | - | - | - | - | - | - | - | - | - | - |
| | Filler 7 | - | - | - | - | 180 | - | - | - | - | - | - | - | - | - | - | - |
| polymerization accelerator (e) | DABE | 0.4 | 0.4 | 0.4 | 0.4 | 0.4 | 0.4 | 0.4 | 0.4 | 0.4 | 0.4 | 0.4 | 0.4 | 0.4 | 0.4 | 0.4 | 0.4 |
| Other | BHT | 0.05 | 0.05 | 0.05 | 0.05 | 0.05 | 0.05 | 0.05 | 0.05 | 0.05 | 0.05 | 0.05 | 0.05 | 0.05 | 0.05 | 0.05 | 0.05 |
| Cavity sealing properties | Without cleaner | 0 | 0 | 2 | 1 | 2 | 2 | 0 | 1 | 2 | 1 | 1 | 2 | 2 | 0 | 0 | 0 |
| | With cleaner | 0 | 0 | 1 | 0 | 1 | 1 | 0 | 0 | 1 | 0 | 1 | 1 | 1 | 0 | 0 | 0 |
| Bond strength to dentin MPa | Without cleaner | 10 | 10 | 6 | 8 | 6 | 7 | 10 | 9 | 6 | 8 | 7 | 6 | 6 | 10 | 10 | 10 |
| | With cleaner | 11 | 11 | 9 | 10 | 9 | 9 | 11 | 10 | 8 | 10 | 8 | 9 | 7 | 11 | 11 | 11 |

**[Table 3]**

| Components (parts by mass) | | Ex. 17 | Ex. 18 | Ex. 19 | Ex. 20 | Ex. 21 | Ex. 22 | Ex. 23 | Ex. 24 | Ex. 25 | Ex. 26 | Ex. 27 | Ex. 28 | Ex. 29 |
|---|---|---|---|---|---|---|---|---|---|---|---|---|---|---|
| Cleaning material | | Cleaning material 3 | Cleaning material 3 | Cleaning material 3 | Cleaning material 1 | Cleaning material 2 | Cleaning material 4 | Cleaning material 5 | Cleaning material 6 | Cleaning material 7 | Cleaning material 8 | Cleaning material 9 | Cleaning material 10 | Cleaning material 11 |
| Monomer (a) having acidic group | MDP | 10 | 10 | 10 | 10 | 10 | 10 | 10 | 10 | 10 | 10 | 10 | 10 | 10 |
| Monomer (b) having no acidic group | Bis-GMA | 30 | 30 | 40 | 30 | 30 | 30 | 30 | 30 | 30 | 30 | 30 | 30 | 30 |
| | D-2.6E | - | - | - | - | - | - | - | - | - | - | - | - | - |
| | 3G | - | - | 50 | 50 | 50 | 50 | 50 | 50 | 50 | 50 | 50 | 50 | 50 |
| | MAEA | 10 | 10 | - | 10 | 10 | 10 | 10 | 10 | 10 | 10 | 10 | 10 | 10 |
| | DD | 50 | 50 | - | - | - | - | - | - | - | - | - | - | - |
| | HEMA | - | - | - | - | - | - | - | - | - | - | - | - | - |
| | DEAA | - | - | - | - | - | - | - | - | - | - | - | - | - |
| Photopolymerization initiator (c-1) | Li-TPO | 0.3 | - | - | - | - | - | - | - | - | - | - | - | - |
| | CQ | 0.2 | 0.2 | 0.2 | 0.2 | 0.2 | 0.2 | 0.2 | 0.2 | 0.2 | 0.2 | 0.2 | 0.2 | 0.2 |
| | BAPO | - | 0.2 | - | - | - | - | - | - | - | - | - | - | - |
| Filler (d) | Filler 1 | 10 | 10 | 10 | 10 | 10 | 10 | 10 | 10 | 10 | 10 | 10 | 10 | 10 |
| | Filler 2 | - | - | - | - | - | - | - | - | - | - | - | - | - |
| | Filler 3 | 250 | 250 | 250 | - | - | - | - | - | - | - | - | - | - |
| | Filler 4 | - | - | - | - | - | - | - | - | - | - | - | - | - |
| | Filler 5 | - | - | - | 180 | 180 | 180 | 180 | 180 | 180 | 180 | 180 | 180 | 180 |
| | Filler 6 | - | - | - | - | - | - | - | - | - | - | - | - | - |
| | Filler 7 | - | - | - | - | - | - | - | - | - | - | - | - | - |
| polymerization accelerator (e) | DABE | 0.4 | 0.4 | 0.4 | 0.4 | 0.4 | 0.4 | 0.4 | 0.4 | 0.4 | 0.4 | 0.4 | 0.4 | 0.4 |
| Other | BHT | 0.05 | 0.05 | 0.05 | 0.05 | 0.05 | 0.05 | 0.05 | 0.05 | 0.05 | 0.05 | 0.05 | 0.05 | 0.05 |
| Cavity sealing properties | Without cleaner | 0 | 2 | 1 | 2 | 2 | 2 | 2 | 2 | 2 | 2 | 2 | 2 | 2 |
| | With cleaner | 0 | 0 | 0 | 1 | 1 | 1 | 1 | 1 | 1 | 1 | 1 | 1 | 1 |
| Bond strength to dentin MPa | Without cleaner | 11 | 6 | 11 | 6 | 6 | 6 | 6 | 6 | 6 | 6 | 6 | 6 | 6 |
| | With cleaner | 13 | 11 | 12 | 8 | 8 | 8 | 8 | 9 | 9 | 8 | 9 | 8 | 8 |

**[Table 4]**

| Components (parts by mass) | | Com. Ex. 1 | Com. Ex. 2 | Com. Ex. 3 |
|---|---|---|---|---|
| Cleaning material | | Cleaning material 3 | Cleaning material 12 | Cleaning material 13 |
| Monomer (a) having acidic group | MDP | - | 10 | 10 |
| Monomer (b) having no acidic group | Bis-GMA | 30 | 30 | 30 |
| | D-2.6E | - | - | - |
| | 3G | 50 | 50 | 50 |
| | DD | 10 | - | - |
| | MAEA | 10 | 10 | 10 |
| Photopolymerization initiator (c-1) | Li-TPO | - | - | - |
| | CQ | 0.2 | 0.2 | 0.2 |
| | BAPO | - | - | - |
| Filler (d) | Filler 1 | 10 | 10 | 10 |
| | Filler 2 | - | - | - |
| | Filler 3 | 250 | - | - |
| | Filler 4 | - | - | - |
| | Filler 5 | - | 180 | 180 |
| | Filler 6 | - | - | - |
| | Filler 7 | - | - | - |
| polymerization accelerator (e) | DABE | 0.4 | 0.4 | 0.4 |
| Other | BHT | 0.05 | 0.05 | 0.05 |
| Cavity sealing properties | Without cleaner | 3 | 2 | 2 |
| | With cleaner | 3 | 3 | 2 |
| Bond strength to dentin MPa | Without cleaner | 0 | 6 | 6 |
| | With cleaner | 0 | 2 | 6 |

As can be seen from the results shown in Tables 2 and 3, the dental filling kits of Examples had a bond strength of 7 MPa or higher on dentin after the tooth surface was cleaned with the dental cleaning material (Y), and the cavity sealing properties had a score of 2 or less when the dental cleaning material (Y) was not used, and a score of 1 or less when the dental cleaning material (Y) was used. Despite being photopolymerizable self-adhesive dental composite resins containing a photopolymerization initiator (c-1), the dental filling kits of Examples showed superior cavity sealing properties with the dental cleaning material (Y) cleaning the interior of cavities, effectively reducing detachment from the cavity floor without causing demineralization of the tooth structure itself while enabling removal of the smear layer.

This is in contrast to Comparative Examples. As shown in Table 4, Comparative Example 1 with the absence of a monomer (a) having an acidic group in the self-adhesive dental composite resin (X) did not show bond strength to dentin. In Comparative Examples 2 and 3 in which the dental cleaning material (Y) did not have a pH in the range of 2.0 or more and less than 9.0, the cavity sealing properties were poor, and did not improve even after the tooth surface was cleaned with the dental cleaning material (Y).

### INDUSTRIAL APPLICABILITY

A dental filling kit of the present invention can be suitably used for filling treatments in dentistry. A dental filling kit of the present invention is particularly suited for the filling treatment of cavities with a depth of 2 mm or more.

## Claims

1. A dental filling kit comprising:
a self-adhesive dental composite resin (X) comprising a monomer (a) having an acidic group, a monomer (b) having no acidic group, a polymerization initiator (c), and a filler (d); and
a dental cleaning material (Y) having a pH of 2.0 or more and less than 9.0.

2. The dental filling kit according to claim 1, wherein the dental cleaning material (Y) comprises a cationic component (f), an anionic component (g), and water (h).

3. The dental filling kit according to claim 2, wherein the anionic component (g) comprises a monomer (g-1) having an acidic group.

4. The dental filling kit according to claim 3, wherein the monomer (g-1) having an acidic group comprises a monomer having a phosphoric acid group.

5. The dental filling kit according to claim 3 or 4, wherein the monomer (g-1) having an acidic group comprises a monomer having a divalent phosphoric acid group with a C6 to C12 alkylene group.

6. The dental filling kit according to any one of claims 3 to 5, wherein the monomer (g-1) having an acidic group comprises a compound identical to the monomer (a) having an acidic group.

7. The dental filling kit according to any one of claims 2 to 6, wherein the cationic component (f) comprises a basic compound.

8. The dental filling kit according to claim 7, wherein the basic compound comprises an organic amine compound (f-1).

9. The dental filling kit according to claim 8, wherein the organic amine compound (f-1) comprises a tertiary organic amine compound.

10. The dental filling kit according to claim 9, wherein the tertiary organic amine compound comprises triethanolamine.

11. The dental filling kit according to any one of claims 2 to 10, wherein the content of the water (h) is 40 to 99.8 parts by mass relative to 100 parts by mass of the dental cleaning material (Y).

12. The dental filling kit according to any one of claims 1 to 11, wherein the dental cleaning material (Y) has a pH of 2.0 or more and less than 8.0.

13. The dental filling kit according to any one of claims 1 to 12, wherein the content of the filler (d) is 50 to 90 parts by mass in total 100 parts by mass of the self-adhesive dental composite resin (X).

14. The dental filling kit according to any one of claims 1 to 13, wherein the dental cleaning material (Y) is essentially free of the monomer (b) having no acidic group.

15. The dental filling kit according to any one of claims 1 to 14, wherein the dental cleaning material (Y) is essentially free of a polymerization initiator.

16. The dental filling kit according to any one of claims 1 to 15, wherein the polymerization initiator (c) comprises a photopolymerization initiator (c-1).
